# EUROPEAN PATENT SPECIFICATION

(11) **EP 4 215 256 B1**
(45) Date of publication and mention of the grant of the patent: **19.11.2025**
(21) Application number: 23152895.1
(22) Date of filing: 23.01.2023
(51) Int. Cl.: B01D 15/18, G01N 30/46, G01N 30/32

(54) **CONTROL METHOD FOR A MUTUALLY INDEPENDENT DUAL ONLINE LIQUID CHROMATOGRAPHY DEVICE**
STEUERUNGSVERFAHREN FÜR VONEINANDER UNABHÄNGIGE DUALE ONLINE-FLÜSSIGKEITSCHROMATOGRAFIEVORRICHTUNG
PROCÉDÉ DE COMMANDE POUR UN DISPOSITIF DE CHROMATOGRAPHIE EN PHASE LIQUIDE EN LIGNE DOUBLE INDÉPENDANT L'UN DE L'AUTRE

(30) Priority: 24.01.2022 KR 20220009849
(43) Date of publication of application: 26.07.2023
(73) Proprietor: Korea University Research and Business Foundation, Seoul 02841 (KR); BERTIS INC., Seongnam-si 13605 (KR)
(72) Inventor: LEE, Sang-Won, 02843 Seoul (KR); KANG, Chaewon, 02474 Seoul (KR)
(74) Representative: V.O.

(56) References cited:
- CN-A- 105 664 527
- CN-U- 209 198 398
- US-A1- 2017 176 400
- WAGNER K ET AL: "AN AUTOMATED ON-LINE MULTIDIMENSIONAL HPLC SYSTEM FOR PROTEIN AND PEPTIDE MAPPING WITH INTEGRATED SAMPLE PREPARATION", ANALYTICAL CHEMISTRY, AMERICAN CHEMICAL SOCIETY, US, vol. 74, no. 4, 15 February 2002 (2002-02-15), pages 809 - 820, XP001115830, ISSN: 0003-2700, DOI: 10.1021/AC010627F

## Description

### BACKGROUND

### 1. Field

The present disclosure relates to a mutually independent dual online liquid chromatography device and a control method thereof.

### 2. Description of the Related Art

In proteomics which is the large-scale study of proteins expressed in living organisms under specific conditions, liquid chromatography with tandem mass spectrometry (LC-MS/MS) is emerging as a very important technology. A bottom-up approach used as a typical example analyzes proteins by hydrolyzing the proteins to smaller pieces, peptides, before analysis using a mass spectrometer.

The bottom-up approach is found to be effective, but the process of hydrolyzing proteins into peptides inevitably causes sample complexity. For example, at least a few tens of millions of peptides are obtained when hydrolyzing proteins expressed from about 20,000 genomes.

Accordingly, to solve the sample complexity issue, it is still necessary to improve efficiency and sensitivity of liquid chromatography.

It is known that sensitivity of liquid chromatography-tandem mass spectrometry (LC-MS/MS) experiment may sharply increase when reducing the inner diameter while keeping the length of the sample separation column constant.

However, the existing liquid chromatography device requires a long time to equilibrate or regenerate columns when hydrophobic media are packed in capillary columns having a large length and a small inner diameter.

In this circumstance, the applicant developed a dual online liquid chromatography device in which an analytic experiment is performed in one of the columns while the other column is being washed and equilibrated to minimize the dead time needed to stop the experiment during column equilibration, thereby speeding up the experiment by about two times.

However, the existing dual online liquid chromatography device employs one valve to which two reverse phase liquid chromatography columns (hereinafter referred to as "sample separation column") are connected together, so when sample analysis is being conducted in the first sample separation column, the second sample separation column is only allowed to be equilibrated. That is, the existing dual online liquid chromatography device fails to completely independently run the two sample separation columns.

Thus, the existing dual online liquid chromatography device needs valve switching after completing the sample separation experiment in the first sample separation column to proceed with the next sample injection onto the second sample separation column, and thus it requires the dead time needed to substantially stop the mass spectrometer for the period of time from the sample injection time until the elution of the sample.

Additionally, to improve the efficiency and sensitivity of the sample separation column, the sample separation column having a large length is used, resulting in increased length over which the sample goes from the sample separation column to the mass spectrometer, and as a consequence, the mass spectrometer has the mass signal dead time during which peptide is not detected while the first peptide is eluted from the sample separation column.

US 2017/176400 A1 discloses a dual online liquid chromatography device comprising a first pump for injecting a first solvent or a mixed solution containing the first solvent and a second solvent; a second pump for injecting the first solvent or the mixed solution containing the first and second solvents; a specimen intake valve connected to the first pump and a specimen injector for injecting a specimen; a column selection valve connected to the second pump and the specimen intake valve; and a dual column valve including a plurality of ports, one side of which is connected to the column selection valve and an opposite side of which is connected to a first column and a second column. The dual column valve may form a first fluid communication with the first column or the first solid phase extraction column, and a second fluid communication with the second column or the second solid phase extraction column, and the dual column valve may selectively have a mode of simultaneously conducting separate analysis of the sample using the first fluid communication, and cleaning and equilibration of the second fluid communication, and a mode of simultaneously conducting separate analysis of the sample using the second fluid communication, and cleaning and equilibration of the first fluid communication.

### [RELATED LITERATURES]

### [Patent Literature]

[Literature 1] Korean Patent Publication No. 10-2015-0112414 (published on October 7, 2015)

### SUMMARY

Accordingly, the present disclosure is directed to providing a mutually independent dual online liquid chromatography device and a control method thereof, in which two sample separation columns can be used completely independently of each other to allow for wash and equilibration, sample injection and isocratic elution of one sample separation column while sample analysis is being conducted in the other sample separation column, thereby eliminating the time necessary for wash and column equilibration, sample injection and initial peptide elution time, thus improving the efficiency of the liquid chromatography-mass spectrometry (LC-MS) experiment as well as the separation of hydrophilic peptides, minimizing the mass signal dead time of the mass spectrometer and maximizing the reverse phase chromatography separation space, thereby greatly improving the peptide detection.

The scope of the invention is defined by the appended claims.

Though not part of the claimed subject-matter, disclosed is a mutually independent dual online liquid chromatography device including a first pump configured to inject a first solvent or a mixed solution containing the first solvent and a second solvent; a second pump configured to inject the first solvent or the mixed solution containing the first solvent and the second solvent; a sample inlet valve to which the first pump and a sample injector configured to inject a sample are connected; a first column valve to which a first sample separation column is connected; a second column valve to which a second sample separation column is connected; and a column selection valve to which the second pump is connected, and interposed between the sample inlet valve, and the first column valve and the second column valve to inject the sample fed from the sample inlet valve onto any one of the first sample separation column and the second sample separation column to separate and analyze the sample in one sample separation column while allowing for wash and equilibration, sample injection and isocratic elution of the other sample separation column.

The sample inlet valve may include a sample inlet port connected to the sample injector; a sample outlet port disposed adjacent to the sample inlet port; a first pump connection port connected to the first pump; a first outlet port disposed adjacent to the first pump connection port and connected to the column selection valve; and a first sample storage loop connection port and a second sample storage loop connection port to which two ends of a sample storage loop are respectively connected, and in a state that the first sample storage loop connection port and the second sample storage loop connection port are connected to the sample inlet port and the sample outlet port, respectively, the sample may be stored in the sample storage loop, and in a state that the first sample storage loop connection port and the second sample storage loop connection port are connected to the first pump connection port and the first outlet port, respectively, the first pump may inject the first solvent onto the first pump connection port to feed the sample stored in the sample storage loop into the column selection valve together with the first solvent.

The first column valve may include a first solid phase extraction column connected to the first sample separation column, the second column valve may include a second solid phase extraction column connected to the second sample separation column, and the column selection valve may include a first inlet port connected to the sample inlet valve; a first column valve connection port connected to the first column valve; a second column valve connection port connected to the second column valve; and a second pump connection port connected to the second pump, and in a state that the sample inlet valve, the first inlet port and the first column valve connection port are connected, the column selection valve may allow for equilibration of the first solid phase extraction column and the first sample separation column, sample injection and isocratic elution, and in a state that the sample inlet valve, the first inlet port and the second column valve connection port are connected, allow for equilibration of the second solid phase extraction column and the second sample separation column, sample injection and isocratic elution.

The first column valve may include a first solid phase extraction column connection port and a first solid phase extraction column transit port connected to two ends of the first solid phase extraction column, respectively; a first solid phase extraction column inlet port connected to the column selection valve, and selectively connected to the first solid phase extraction column connection port and the first solid phase extraction column transit port; a first sample separation column connection port connected to the first sample separation column, and connected to or disconnected from the first solid phase extraction column connection port; and a second outlet port disposed adjacent to the first solid phase extraction column transit port and connected to or disconnected from the first solid phase extraction column transit port, and the second column valve may include a second solid phase extraction column connection port and a second solid phase extraction column transit port connected to two ends of the second solid phase extraction column, respectively; a second solid phase extraction column inlet port connected to the column selection valve, and selectively connected to the second solid phase extraction column connection port and the second solid phase extraction column transit port; a second sample separation column connection port connected to the second sample separation column, and connected to or disconnected from the second solid phase extraction column connection port; and a third outlet port disposed adjacent to the second solid phase extraction column transit port and connected to or disconnected from the second solid phase extraction column transit port.

Also disclosed is a method for controlling a mutually independent dual online liquid chromatography device including (A) storing a sample in a sample storage loop of a sample inlet valve, and at the same time, equilibrating a first sample separation column of a first column valve and a second sample separation column of a second column valve; (B) performing isocratic elution from the first sample separation column, and at the same time, continuously equilibrating the second sample separation column; (C) performing valve switching of a column selection valve to perform gradient elution from the first sample separation column and separation and analysis of the sample, and at the same time, perform equilibration of the second sample separation column, sample injection and isocratic elution; (D) completing the separation and analysis of the sample in the first sample separation column and the isocratic elution from the second sample separation column at the same time; (E) performing valve switching of the column selection valve to perform gradient elution from the second sample separation column and separation and analysis of the sample, and at the same time, performing wash and equilibration of the first sample separation column, sample injection and isocratic elution; (F) completing the separation and analysis of the sample in the second sample separation column and the isocratic elution from the first sample separation column at the same time; and (G) performing the steps (C) to (F) in a sequential order repeatedly according to the number of times of separation and analysis of the sample or the number of samples, wherein the step (C) includes washing the second sample separation column before the equilibration of the second sample separation column, the sample injection and the isocratic elution.

The step (A) may include (A-1) injecting and storing, by a sample injector connected to the sample inlet valve, the sample in the sample storage loop; (A-2) injecting, by a first pump connected to the sample inlet valve, a first solvent onto the first solid phase extraction column and the first sample separation column of the first column valve via the column selection valve to equilibrate the first solid phase extraction column and the first sample separation column; and (A-3) injecting, by a second pump connected to the column selection valve, the first solvent onto the second solid phase extraction column and the second sample separation column of the second column valve to equilibrate the second solid phase extraction column and the second sample separation column.

The sample inlet valve may include a sample inlet port connected to the sample injector; a sample outlet port disposed adjacent to the sample inlet port; a first pump connection port connected to the first pump; a first outlet port disposed adjacent to the first pump connection port and connected to the column selection valve; and a first sample storage loop connection port and a second sample storage loop connection port to which two ends of the sample storage loop are connected respectively, and the step (A-1) may include storing the sample in the sample storage loop in a state that the first sample storage loop connection port and the second sample storage loop connection port are connected to the sample inlet port and the sample outlet port, respectively.

The column selection valve may include a first inlet port connected to the first outlet port; a first column valve connection port connected to the first column valve; a second column valve connection port connected to the second column valve; and a second pump connection port connected to the second pump, the step (A-2) may include, in a state that the first pump connection port, the first outlet port, the first inlet port and the first column valve connection port are connected, injecting, by the first pump, the first solvent onto the first solid phase extraction column and the first sample separation column to equilibrate the first solid phase extraction column and the first sample separation column, and the step (A-3) may include, in a state that the second pump connection port and the second column valve connection port are connected, injecting, by the second pump, the first solvent onto the second solid phase extraction column and the second sample separation column to equilibrate the second solid phase extraction column and the second sample separation column.

The first column valve may include a first solid phase extraction column connection port and a first solid phase extraction column transit port connected to two ends of the first solid phase extraction column, respectively; a first solid phase extraction column inlet port connected to the first column valve connection port, and selectively connected to the first solid phase extraction column connection port and the first solid phase extraction column transit port; a first sample separation column connection port connected to the first sample separation column, and connected to or disconnected from the first solid phase extraction column connection port; and a second outlet port disposed adjacent to the first solid phase extraction column transit port and connected to or disconnected from the first solid phase extraction column transit port, and the second column valve may include a second solid phase extraction column connection port and a second solid phase extraction column transit port connected to two ends of the second solid phase extraction column, respectively; a second solid phase extraction column inlet port connected to the second column valve connection port, and selectively connected to the second solid phase extraction column connection port and the second solid phase extraction column transit port; a second sample separation column connection port connected to the second sample separation column, and connected to or disconnected from the second solid phase extraction column connection port; and a third outlet port disposed adjacent to the second solid phase extraction column transit port and connected to or disconnected from the second solid phase extraction column transit port, and the method may further include, after the step (A), in a state that the first solid phase extraction column inlet port and the first solid phase extraction column connection port are connected and the first solid phase extraction column transit port and the second outlet port are connected, injecting the sample stored in the sample storage loop onto the first solid phase extraction column together with the first solvent fed from the first pump and desalting and concentrating the sample; and in a state that the second solid phase extraction column inlet port and the second solid phase extraction column transit port are connected and the second solid phase extraction column connection port and the second sample separation column connection port are connected, injecting, by the second pump, the first solvent onto the second solid phase extraction column and the second sample separation column to continuously equilibrate the second solid phase extraction column and the second sample separation column.

The step (B) may include, in a state that the first solid phase extraction column inlet port and the first solid phase extraction column transit port are connected and the first solid phase extraction column connection port and the first sample separation column connection port are connected, injecting, by the first pump, a mixed solution containing the first solvent and a second solvent onto the first solid phase extraction column and the first sample separation column and performing the isocratic elution.

The step (C) may include (C-1) injecting, by the second pump, the mixed solution containing the increasing second solvent over time onto the first solid phase extraction column and the first sample separation column to perform gradient elution and separation and analysis of the sample, and at the same time, injecting, by the first pump, the first solvent onto the second solid phase extraction column and the second sample separation column to continuously equilibrate the second solid phase extraction column and the second sample separation column and store the sample in the sample storage loop; (C-2) during the separation and analysis of the sample in the first sample separation column, injecting, by the first pump, the first solvent onto the sample storage loop to inject the sample stored in the sample storage loop onto the second solid phase extraction column and desalt and concentrate the sample; and (C-3) during the separation and analysis of the sample in the first sample separation column, injecting, by the first pump, the mixed solution containing the first solvent and the second solvent onto the second solid phase extraction column and the second sample separation column to perform the isocratic elution.

The step (C-1) may include, in a state that the second pump connection port and the first column valve connection port are connected, the first solid phase extraction column inlet port and the first solid phase extraction column transit port are connected and the first solid phase extraction column connection port and the first sample separation column connection port are connected, injecting, by the second pump, the mixed solution containing the increasing second solvent over time onto the first solid phase extraction column and the first sample separation column to perform gradient elution and separation and analysis of the sample, and at the same time, in a state that the first pump connection port and the first outlet port are connected, the first inlet port and the second column valve connection port are connected, the second solid phase extraction column inlet port and the second solid phase extraction column transit port are connected and the second solid phase extraction column connection port and the second sample separation column connection port are connected, injecting, by the first pump, the first solvent onto the second solid phase extraction column and the second sample separation column to continuously equilibrate the second solid phase extraction column and the second sample separation column, and at the same time, in a state that the first sample storage loop connection port and the second sample storage loop connection port are connected to the sample inlet port and the sample outlet port, respectively, receiving the sample from the sample injector and injecting and storing the sample in the sample storage loop, the step (C-2) may include, in a state that the first pump connection port, the first sample storage loop connection port, the second sample storage loop connection port and the first outlet port are connected, the first inlet port and the second column valve connection port are connected, the second solid phase extraction column inlet port and the second solid phase extraction column connection port are connected and the second solid phase extraction column transit port and the third outlet port are connected, injecting the sample stored in the sample storage loop onto the second solid phase extraction column together with the first solvent fed from the first pump and desalting and concentrating the sample, and the step (C-3) may include, in a state that the first pump connection port and the first outlet port are connected, the first inlet port and the second column valve connection port are connected, the second solid phase extraction column inlet port and the second solid phase extraction column transit port are connected and the second solid phase extraction column connection port and the second sample separation column connection port are connected, injecting, by the first pump, the mixed solution containing the first solvent and the second solvent onto the second solid phase extraction column and the second sample separation column to perform the isocratic elution.

When the step (G) includes repeatedly performing the steps (C) to (F) in a sequential order, the step (C-1) may include, before equilibrating the second solid phase extraction column and the second sample separation column, in a state that the first pump connection port and the first outlet port are connected and the first inlet port and the second column valve connection port are connected, injecting, by the first pump, the mixed solution containing the first solvent and the second solvent onto the second solid phase extraction column and the second sample separation column to wash the second solid phase extraction column and the second sample separation column.

The step (D) may include matching an end time of the separation and analysis of the sample in the first sample separation column with a time immediately before a first peptide is eluted from the second sample separation column.

The step (E) may include (E-1) feeding, by the second pump, the mixed solution containing the increasing second solvent over time into the second solid phase extraction column and the second sample separation column and performing gradient elution and separation and analysis of the sample, and at the same time, injecting, by the first pump, the mixed solution containing the first solvent and the second solvent onto the first solid phase extraction column and the first sample separation column to wash the first solid phase extraction column and the first sample separation column, and feeding the first solvent into the first solid phase extraction column and the first sample separation column to continuously equilibrate the first solid phase extraction column and the first sample separation column and store the sample in the sample storage loop; (E-2) during the separation and analysis of the sample in the second sample separation column, injecting, by the first pump, the first solvent onto the sample storage loop to inject the sample stored in the sample storage loop onto the first solid phase extraction column and desalt and concentrate the sample; and (E-3) during the separation and analysis of the sample in the second sample separation column, feeding, by the first pump, the mixed solution containing the first solvent and the second solvent into the first solid phase extraction column and the first sample separation column to perform the isocratic elution.

The step (E-1) may include, in a state that the second pump connection port and the second column valve connection port are connected, the second solid phase extraction column inlet port and the second solid phase extraction column transit port are connected and the second solid phase extraction column connection port and the second sample separation column connection port are connected, feeding, by the second pump, the mixed solution containing the increasing second solvent over time into the second solid phase extraction column and the second sample separation column to perform gradient elution and separation and analysis of the sample, and at the same time, in a state that the first pump connection port and the first outlet port are connected, the first inlet port and the first column valve connection port are connected, the first solid phase extraction column inlet port and the first solid phase extraction column transit port are connected and the first solid phase extraction column connection port and the first sample separation column connection port are connected, injecting, by the first pump, the mixed solution containing the first solvent and the second solvent onto the first solid phase extraction column and the first sample separation column to wash the first solid phase extraction column and the first sample separation column and injecting the first solvent onto the first solid phase extraction column and the first sample separation column to equilibrate the first solid phase extraction column and the first sample separation column, and at the same time, in a state that the first sample storage loop connection port and the second sample storage loop connection port are connected to the sample inlet port and the sample outlet port, respectively, receiving the sample from the sample injector and injecting and storing the sample in the sample storage loop, the step (E-2) may include, in a state that the first pump connection port, the first sample storage loop connection port, the second sample storage loop connection port and the first outlet port are connected, the first inlet port and the first column valve connection port are connected, the first solid phase extraction column inlet port and the first solid phase extraction column connection port are connected and the first solid phase extraction column transit port and the second outlet port are connected, injecting the sample stored in the sample storage loop onto the first solid phase extraction column together with the first solvent fed from the first pump and desalting and concentrating the sample, and the step (E-3) may include, in a state that the first pump connection port and the first outlet port are connected, the first inlet port and the first column valve connection port are connected, the first solid phase extraction column inlet port and the first solid phase extraction column transit port are connected and the first solid phase extraction column connection port and the first sample separation column connection port are connected, injecting, by the first pump, the mixed solution containing the first solvent and the second solvent onto the first solid phase extraction column and the first sample separation column and performing the isocratic elution.

The step (F) may include matching an end time of the separation and analysis of the sample in the second sample separation column with a time immediately before the first peptide is eluted from the first sample separation column.

According to an embodiment of the present disclosure, it is possible to achieve continuous analysis without the influence of the dead time needed to stop the mass spectrometer during column equilibration by washing and equilibrating the second sample separation column while separating and analyzing the sample in the first sample separation column.

Additionally, according to an embodiment of the present disclosure, since the first solid phase extraction column and the first sample separation column, and the second solid phase extraction column and the second sample separation column are mounted in the first column valve having 6 ports and the second column valve having 6 ports, respectively, it is possible to allow for sample injection onto the second solid phase extraction column by switching the second column valve while the sample is separated and analyzed in the first sample separation column of the first column valve, thereby achieving continuous analysis without the influence of the dead time for sample injection and achieving fast sample injection, thus maximizing analysis efficiency.

Additionally, according to an embodiment of the present disclosure, after the sample is injected onto the second solid phase extraction column while the sample is separated and analyzed in the first sample separation column, the second column valve is switched again to allow for isocratic elution of the sample injected onto the second solid phase extraction column through the second sample separation column for the time required for the sample to go from the long sample separation column until immediately before the mass spectrometer, to match the time immediately before the end of separation and analysis in the first sample separation column with the time at which the first peptide is eluted from the second sample separation column, so that separation and analysis in the second sample separation column starts as soon as separation and analysis in the first sample separation column ends, thereby minimizing the mass signal dead time of the mass spectrometer and maximizing the reverse phase chromatography separation space, thus improving the separation performance of hydrophilic samples.

Additionally, according to an embodiment of the present disclosure, the automatic switching valve can automate all the experimental processes, thereby maximize reproducibility of experiments.

### BRIEF DESCRIPTION OF THE DRAWINGS

FIG. 1 is a graph illustrating the operation of a mutually independent dual online liquid chromatography device according to the present disclosure.
FIG. 2 is a graph illustrating the improved separation performance of hydrophilic samples by a mutually independent dual online liquid chromatography device according to the present disclosure.
FIGS. 3 to 11 are operational diagrams of a mutually independent dual online liquid chromatography device according to the present disclosure.

### DETAILED DESCRIPTION

To fully understand the present disclosure, the operational advantages of the present disclosure and the objectives achieved by the embodiment of the present disclosure, reference is made to the accompanying drawings depicting an exemplary embodiment of the present disclosure and the description in the accompanying drawings.

Hereinafter, an exemplary embodiment of the present disclosure will be described with reference to the accompanying drawings to provide a detailed description of the present disclosure. Like reference signs presented in each drawing denote like elements.

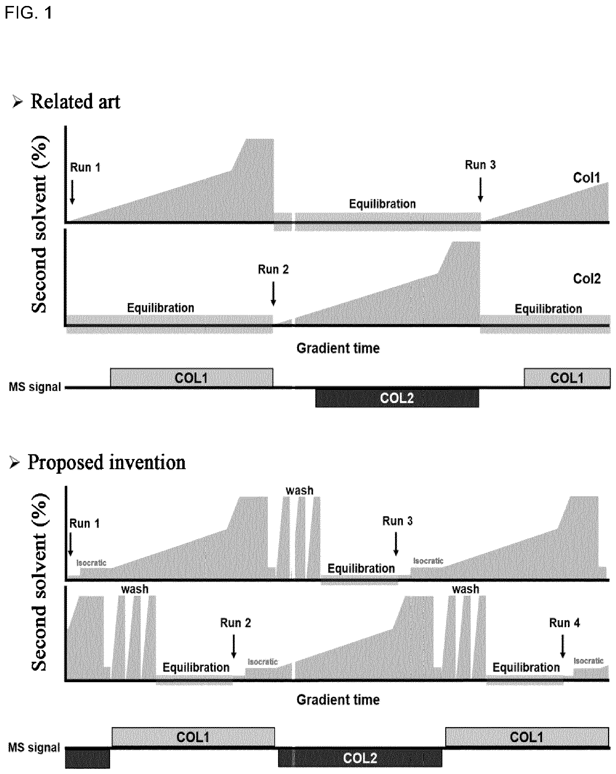

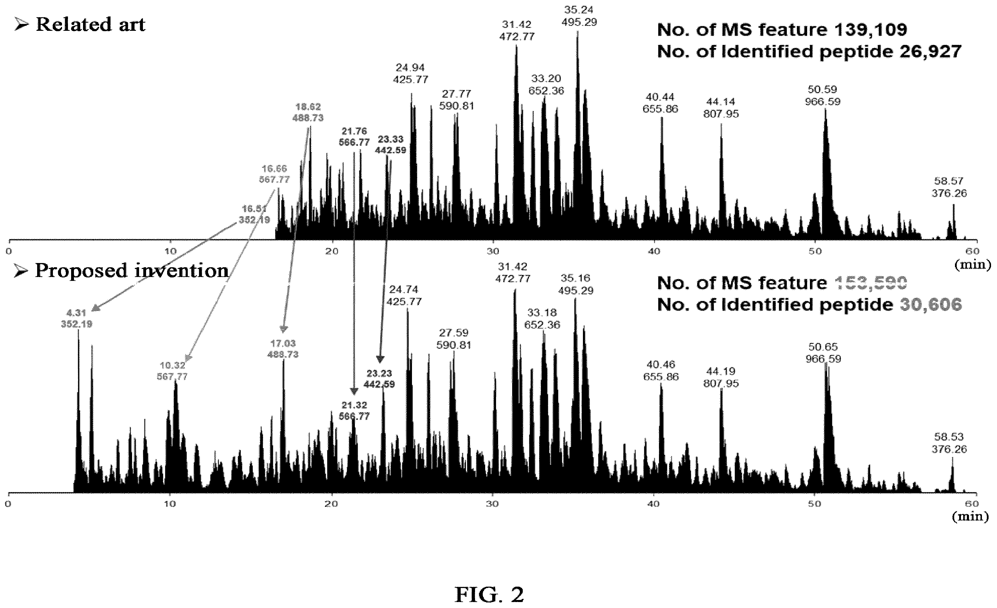

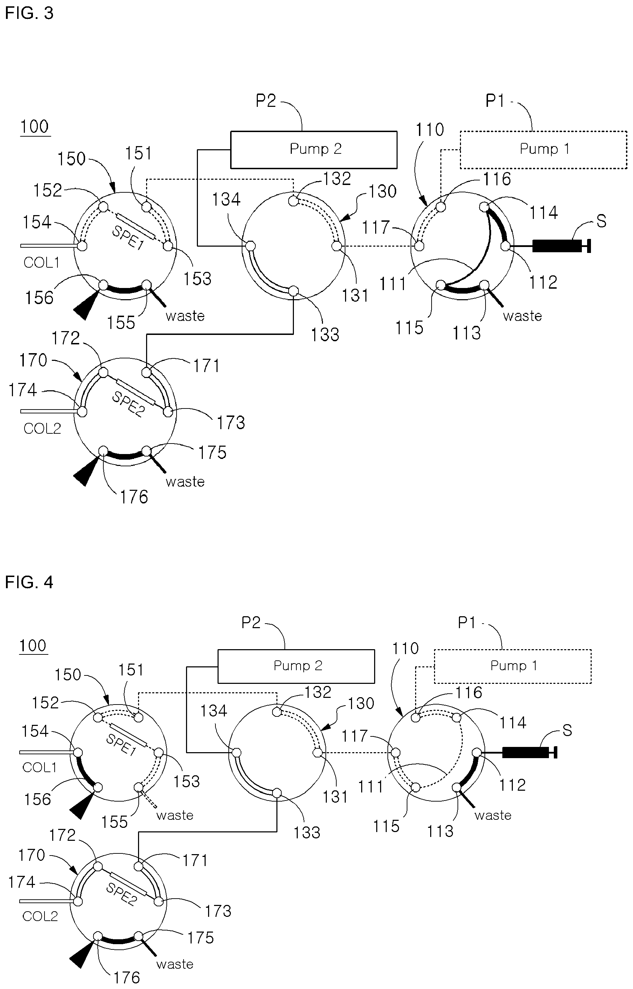

FIG. 1 is a graph illustrating the operation of a mutually independent dual online liquid chromatography device according to the present disclosure, FIG. 2 is a graph illustrating the improved separation performance of hydrophilic samples by the mutually independent dual online liquid chromatography device according to the present disclosure, and FIGS. 3 to 11 are operational diagrams of a mutually independent dual online liquid chromatography device according to the present disclosure.

Referring to FIGS. 1 to 11, the mutually independent dual online liquid chromatography device 100 according to the present disclosure includes a first pump P1 to inject a first solvent or a mixed solution containing the first solvent and a second solvent, a second pump P2 to inject the first solvent or the mixed solution containing the first solvent and the second solvent, a sample inlet valve 110 to which the first pump P1 and a sample injector S configured to inject a sample are connected, a first column valve 150 to which a first reverse phase liquid chromatography column (hereinafter referred to as a "first sample separation column COL1") is connected and including a first solid phase extraction column SPE1 which is connected to the first sample separation column COL1, a second column valve 170 to which a second reverse phase liquid chromatography column (hereinafter referred to as a "second sample separation column COL2") is connected and including a second solid phase extraction column SPE2 which is connected to the second sample separation column COL2, and a column selection valve 130 connected to the sample inlet valve 110, the first column valve 150 and the second column valve 170.

Each of the first pump P1 and the second pump P2 is a pump used to inject the first solvent or the mixed solution containing the first solvent and the second solvent, and the first solvent may be a hydrophilic solvent, for example, 0.1% formic acid in water, and the second solvent may be a hydrophobic solvent, for example, a solution containing 0.1% formic acid and 99.9% acetonitrile.

The sample inlet valve 110 serves to receive the sample from the sample injector S and store it in a sample storage loop 111, and then receive the first solvent from the first pump P1 and feed the sample stored in the sample storage loop 111 into the column selection valve 130.

The sample inlet valve 110 includes a 6-port automatic switching valve having 6 ports.

The sample inlet valve 110 includes a sample inlet port 112 connected to the sample injector S, a sample outlet port 113 disposed adjacent to the sample inlet port 112, a first pump connection port 116 connected to the first pump P1, a first outlet port 117 disposed adjacent to the first pump connection port 116 and connected to the column selection valve 130, and a first sample storage loop connection port 114 and a second sample storage loop connection port 115 to which two ends of the sample storage loop 111 are respectively connected.

In a state that the first sample storage loop connection port 114 and the second sample storage loop connection port 115 respectively connected to the two ends of the sample storage loop 111 are connected to the sample inlet port 112 and the sample outlet port 113, respectively, the sample is fed into the sample inlet port 112 and continuously stored in the sample storage loop 111 through the sample injector S.

Additionally, in a state that the first sample storage loop connection port 114 and the second sample storage loop connection port 115 are connected to the first pump connection port 116 and the first outlet port 117, respectively, the first solvent is injected into a first pump inlet port and the sample stored in the sample storage loop 111 is continuously fed into the column selection valve 130 through the first pump P1.

The column selection valve 130 serves to feed the sample fed from the sample inlet valve 110 into the first solid phase extraction column SPE1 and the first sample separation column COL1 connected in the first column valve 150, and the second solid phase extraction column SPE2 and the second sample separation column COL2 connected in the second column valve 170 as described below.

Additionally, the column selection valve 130 injects the sample fed from the sample inlet valve 110 onto any one of the first sample separation column COL1 and the second sample separation column COL2 to separate and analyze the sample in one sample separation column while allowing for column wash and equilibration, sample injection and desalting, and isocratic elution in the other sample separation column.

The column selection valve 130 includes a 4-port automatic switching valve having 4 ports.

The column selection valve 130 includes a first inlet port 131 connected to the first outlet port 117, a first column valve connection port 132 connected to the first column valve 150, a second column valve connection port 133 connected to the second column valve 170, and a second pump connection port 134 connected to the second pump P2.

The column selection valve 130 may be connected to each of the second pump P2 and the sample inlet valve 110 to which the first pump P1 is connected, to selectively introduce the first solvent or the sample or the mixed solution containing the first solvent and the second solvent together with the first solvent. The first solvent or the sample or the mixed solution containing the first solvent and the second solvent together with the first solvent introduced into the column selection valve 130 is fed into the first column valve 150 to allow for wash and equilibration, sample injection and desalting and isocratic elution and gradient elution of the first solid phase extraction column SPE1 and the first sample separation column COL1, and is also fed into the second column valve 170 to allow for wash and equilibration, sample injection and desalting and isocratic elution and gradient elution of the second solid phase extraction column SPE2 and the second sample separation column COL2.

That is, in a state that the first outlet port 117, the first inlet port 131 and the first column valve connection port 132 are connected, the column selection valve 130 allows for wash and equilibration, sample injection and desalting and isocratic elution and gradient elution of the first solid phase extraction column SPE1 and the first sample separation column COL1, and in a state that the first outlet port 117, the first inlet port 131 and the second column valve connection port 133 are connected, the column selection valve 130 allows for wash and equilibration, sample injection and desalting and isocratic elution and gradient elution of the second solid phase extraction column SPE2 and the second sample separation column COL2.

The first column valve 150 allows for sample injection and desalting of the first solid phase extraction column SPE1, injection of the sample injected onto the first solid phase extraction column SPE1 onto the first sample separation column COL1 through valve switching, and isocratic elution and gradient elution to separate and analyze the sample. Additionally, the second column valve 170 allows for sample injection and desalting of the second solid phase extraction column SPE2, injection of the sample injected onto the second solid phase extraction column SPE2 onto the second sample separation column COL2 through valve switching, and isocratic elution and gradient elution to separate and analyze the sample.

Each of the first column valve 150 and the second column valve 170 includes a 6-port automatic switching valve having 6 ports.

The first column valve 150 includes a first solid phase extraction column connection port 152 and a first solid phase extraction column transit port 153 connected to two ends of the first solid phase extraction column SPE1, respectively, a first solid phase extraction column inlet port 151 connected to the first column valve connection port 132 and selectively connected to the first solid phase extraction column connection port 152 and the first solid phase extraction column transit port 153, a first sample separation column connection port 154 connected to the first sample separation column COL1 and connected to or disconnected from the first solid phase extraction column connection port 152, a second outlet port 155 disposed adjacent to the first solid phase extraction column transit port 153 and connected to or disconnected from the first solid phase extraction column transit port 153, and a first column outlet port 156 disposed adjacent to the first sample separation column connection port 154 and selectively connected to the first sample separation column connection port 154 and the second outlet port 155.

The second column valve 170 includes a second solid phase extraction column connection port 172 and a second solid phase extraction column transit port 173 connected to two ends of the second solid phase extraction column SPE2, respectively, a second solid phase extraction column inlet port 171 connected to the second column valve connection port 133 and selectively connected to the second solid phase extraction column connection port 172 and the second solid phase extraction column transit port 173, a second sample separation column connection port 174 connected to the second sample separation column COL2 and connected to or disconnected from the second solid phase extraction column connection port 172, a third outlet port 175 disposed adjacent to the second solid phase extraction column transit port 173 and connected to or disconnected from the second solid phase extraction column transit port 173, and a second column outlet port 176 disposed adjacent to the second sample separation column connection port 174 and selectively connected to the second sample separation column connection port 174 and the third outlet port 175.

The first column valve 150 and the second column valve 170 are separately provided to inject the sample onto any one of the first sample separation column COL1 and the second sample separation column COL2 while separating and analyzing the sample in the other one. Additionally, each of the first column valve 150 and the second column valve 170 includes a 6-port automatic switching valve to allow for column wash and equilibration, sample injection, desalting and isocratic elution of any one of the first sample separation column COL1 and the second sample separation column COL2 while sample analysis is being conducted in the other one, impossible for the existing dual online liquid chromatography device to perform.

This device performs wash and equilibration of the second sample separation column COL2 during the separation and analysis of the sample in the first sample separation column COL1, thereby achieving continuous analysis without the influence of the dead time needed to stop the mass spectrometer (MS) during the wash and equilibration of the column. Additionally, this device allows for injection of the sample onto the second solid phase extraction column SPE2 during the separation and analysis of the sample in the first sample separation column COL1, thereby achieving continuous analysis without the influence of the dead time for sample injection.

Additionally, this device allows for injection of the sample onto the second solid phase extraction column SPE2 during separation and analysis of the sample in the first sample separation column COL1 and then isocratic elution of the sample injected onto the second solid phase extraction column SPE2 from the second sample separation column COL2, so that separation and analysis in the second sample separation column COL2 starts as soon as separation and analysis in the first sample separation column COL1 ends, thereby minimizing the mass signal dead time of the mass spectrometer (MS) and improving the separation performance of hydrophilic peptides.

A method for controlling the separation and analysis of the sample using the mutually independent dual online liquid chromatography device 100 according to the present disclosure as described above will be described as follows.

Referring to FIG. 3, the method for controlling the mutually independent dual online liquid chromatography device 100 starts with initial mode operation.

In the initial mode, the sample is stored in the sample storage loop 111 of the sample inlet valve 110, and at the same time, equilibration is performed on the first solid phase extraction column SPE1 and the first sample separation column COL1 of the first column valve 150 and the second solid phase extraction column SPE2 and the second sample separation column COL2 of the second column valve 170.

That is, the sample injector S connected to the sample inlet valve 110 injects and stores the sample in the sample storage loop 111. In a state that the first sample storage loop connection port 114 and the second sample storage loop connection port 115 are connected to the sample inlet port 112 and the sample outlet port 113, respectively, the sample is fed from the sample injector S and injected and stored in the sample storage loop 111.

Additionally, the first pump P1 connected to the sample inlet valve 110 injects the first solvent onto the first solid phase extraction column SPE1 and the first sample separation column COL1 of the first column valve 150 via the column selection valve 130 to equilibrate the first solid phase extraction column SPE1 and the first sample separation column COL1. Specifically, in a state that the first pump connection port 116 and the first outlet port 117 are connected, the first pump P1 feeds the first solvent into the column selection valve 130. Additionally, in a state that the first inlet port 131 and the first column valve connection port 132 are connected, the first solvent fed into the column selection valve 130 is fed into the first column valve 150. Additionally, in a state that the first solid phase extraction column inlet port 151 and the first solid phase extraction column transit port 153 are connected and the first solid phase extraction column connection port 152 and the first sample separation column connection port 154 are connected, the first solvent fed into the first column valve 150 is injected onto the first solid phase extraction column SPE1 and the first sample separation column COL1 to equilibrate the first solid phase extraction column SPE1 and the first sample separation column COL1.

Additionally, the second pump P2 connected to the column selection valve 130 injects the first solvent onto the second solid phase extraction column SPE2 and the second sample separation column COL2 of the second column valve 170 to equilibrate the second solid phase extraction column SPE2 and the second sample separation column COL2. Specifically, in a state that the second pump connection port 134 and the second column valve connection port 133 are connected, the second pump P2 feeds the first solvent into the second column valve 170. Additionally, in a state that the second solid phase extraction column inlet port 171 and the second solid phase extraction column transit port 173 are connected and the second solid phase extraction column connection port 172 and the second sample separation column connection port 174 are connected, the first solvent fed into the second column valve 170 is injected onto the second solid phase extraction column SPE2 and the second sample separation column COL2 to equilibrate the second solid phase extraction column SPE2 and the second sample separation column COL2.

Additionally, after the initial mode, referring to FIG. 4, the method for controlling the mutually independent dual online liquid chromatography device 100 carries out a first mode.

In the first mode, during injection of the sample stored in the sample storage loop 111 onto the first solid phase extraction column SPE1 and desalting and concentrating, the second solid phase extraction column SPE2 and the second sample separation column COL2 are continuously equilibrated.

That is, in a state that the first solid phase extraction column inlet port 151 and the first solid phase extraction column connection port 152 are connected and the first solid phase extraction column transit port 153 and the second outlet port 155 are connected, the sample stored in the sample storage loop 111 is injected onto the first solid phase extraction column SPE1 together with the first solvent fed from the first pump P1, and is desalted and concentrated. Specifically, in a state that the first pump connection port 116 and the first sample storage loop connection port 114 are connected and the second sample storage loop connection port 115 and the first outlet port 117 are connected, the sample stored in the sample storage loop 111 is fed into the column selection valve 130 together with the first solvent fed from the first pump P1. Additionally, in a state that the first inlet port 131 and the first column valve connection port 132 are connected, the first solvent and the sample fed into the column selection valve 130 are fed into the first column valve 150. Additionally, in a state that the first solid phase extraction column inlet port 151 and the first solid phase extraction column connection port 152 are connected and the first solid phase extraction column transit port 153 and the second outlet port 155 are connected, the first solvent and the sample fed into the first column valve 150 are injected onto the first solid phase extraction column SPE1. Additionally, the sample is desalted and concentrated in the first solid phase extraction column SPE1 by additionally injecting the first solvent, for example, in an amount of about 2 µL to cause salts to melt in the first solvent which is a water-soluble solvent and exit the second outlet port 155.

Additionally, in a state that the second solid phase extraction column inlet port 171 and the second solid phase extraction column transit port 173 are connected and the second solid phase extraction column connection port 172 and the second sample separation column connection port 174 are connected, the second pump P2 injects the first solvent onto the second solid phase extraction column SPE2 and the second sample separation column COL2 to continuously equilibrate the second solid phase extraction column SPE2 and the second sample separation column COL2. Specifically, in a state that the second pump connection port 134 and the second column valve connection port 133 are connected, the second pump P2 feeds the first solvent into the second column valve 170. Additionally, in a state that the second solid phase extraction column inlet port 171 and the second solid phase extraction column transit port 173 are connected and the second solid phase extraction column connection port 172 and the second sample separation column connection port 174 are connected, the first solvent fed into the second column valve 170 is injected onto the second solid phase extraction column SPE2 and the second sample separation column COL2 to continuously equilibrate the second solid phase extraction column SPE2 and the second sample separation column COL2.

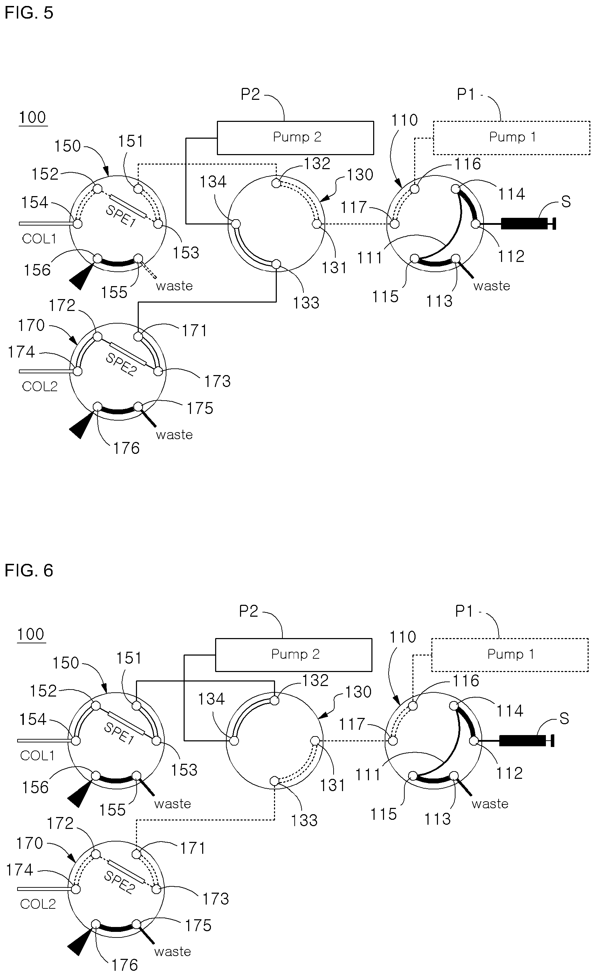

Additionally, after the first mode, referring to FIG. 5, the method for controlling the mutually independent dual online liquid chromatography device 100 carries out a second mode.

In the second mode, during isocratic elution from the first solid phase extraction column SPE1 and the first sample separation column COL1, the second solid phase extraction column SPE2 and the second sample separation column COL2 are continuously equilibrated.

That is, in a state that the first solid phase extraction column inlet port 151 and the first solid phase extraction column transit port 153 are connected and the first solid phase extraction column connection port 152 and the first sample separation column connection port 154 are connected, the first pump P1 injects the mixed solution containing the first solvent and the second solvent onto the first solid phase extraction column SPE1 and the first sample separation column COL1 to perform isocratic elution. The mixed solution used in the isocratic elution may be a mixture of 90% the first solvent and 10% the second solvent, but the scope of protection of the present disclosure is not limited thereto and the mixed solution used in the isocratic elution may have varying ratios of the first solvent and the second solvent as necessary.

Specifically, in a state that the first pump connection port 116 and the first outlet port 117 are connected, the first pump P1 feeds the mixed solution containing the first solvent and the second solvent into the column selection valve 130. Additionally, in a state that the first inlet port 131 and the first column valve connection port 132 are connected, the mixed solution containing the first solvent and the second solvent fed into the column selection valve 130 is fed into the first column valve 150. Additionally, in a state that the first solid phase extraction column inlet port 151 and the first solid phase extraction column transit port 153 are connected and the first solid phase extraction column connection port 152 and the first sample separation column connection port 154 are connected, the mixed solution containing the first solvent and the second solvent fed into the first column valve 150 is injected onto the first solid phase extraction column SPE1 and the first sample separation column COL1 to perform isocratic elution.

The dissociation level of the sample injected onto the first solid phase extraction column SPE1 increases by the isocratic elution, and the dissociated hydrophilic peptide is introduced into the first sample separation column COL1. The isocratic elution process is performed for the time required for the first peptide to go from the first sample separation column COL1 until immediately before the mass spectrometer (MS). Accordingly, since a data collection signal is sent to the mass spectrometer (MS) immediately before the first peptide is eluted from the first sample separation column COL1 by the mass spectrometer (MS), it is possible to minimize the mass signal dead time during which any peptide is not detected in the mass spectrometer (MS). Additionally, as shown in FIG. 2, it is possible to maximize the reverse phase chromatography separation space of hydrophilic peptides by the isocratic elution.

Additionally, in a state that the second solid phase extraction column inlet port 171 and the second solid phase extraction column transit port 173 are connected and the second solid phase extraction column connection port 172 and the second sample separation column connection port 174 are connected, the second pump P2 injects the first solvent onto the second solid phase extraction column SPE2 and the second sample separation column COL2 to continuously equilibrate the second solid phase extraction column SPE2 and the second sample separation column COL2. Specifically, in a state that the second pump connection port 134 and the second column valve connection port 133 are connected, the second pump P2 feeds the first solvent into the second column valve 170.

Additionally, in a state that the second solid phase extraction column inlet port 171 and the second solid phase extraction column transit port 173 are connected and the second solid phase extraction column connection port 172 and the second sample separation column connection port 174 are connected, the first solvent fed into the second column valve 170 is injected onto the second solid phase extraction column SPE2 and the second sample separation column COL2 to continuously equilibrate the second solid phase extraction column SPE2 and the second sample separation column COL2.

Additionally, after the second mode, referring to FIGS. 6 to 8, the method for controlling the mutually independent dual online liquid chromatography device 100 carries out a third mode.

In the third mode, during gradient elution from the first sample separation column COL1 and separation and analysis of the sample, equilibration of the second solid phase extraction column SPE2 and the second sample separation column COL2, sample injection and isocratic elution are performed.

That is, when the isocratic elution from the first solid phase extraction column SPE1 and the first sample separation column COL1 is completed, the second pump P2 feeds the mixed solution containing the increasing second solvent over time into the first solid phase extraction column SPE1 and the first sample separation column COL1 to perform gradient elution and separation and analysis of the sample, and at the same time, the first pump P1 injects the first solvent onto the second solid phase extraction column SPE2 and the second sample separation column COL2 to continuously equilibrate the second solid phase extraction column SPE2 and the second sample separation column COL2 and stores the sample in the sample storage loop 111.

Specifically, as shown in FIG. 6, in a state that the second pump connection port 134 and the first column valve connection port 132 are connected, the second pump P2 feeds the mixed solution containing the first solvent and the second solvent into the first column valve 150. Additionally, in a state that the first solid phase extraction column inlet port 151 and the first solid phase extraction column transit port 153 are connected and the first solid phase extraction column connection port 152 and the first sample separation column connection port 154 are connected, the mixed solution fed into the first column valve 150 is injected onto the first solid phase extraction column SPE1 and the first sample separation column COL1 to perform gradient elution and separation and analysis of the sample. As shown in FIG. 1, the gradient elution is performed with varying ratios of the first solvent and the second solvent in the mixed solution containing the first solvent and the second solvent over time using the second pump P2. That is, the initial condition of the mixed solution starts from 90% the first solvent and 10% the second solvent in the same way as the isocratic elution condition, and as the dissociation level of the sample increases with the increasing proportion of the second solvent in the mixed solution, the sample injected onto the first solid phase extraction column SPE1 is introduced into the first sample separation column COL1, and subsequently, analysis is conducted through separation of the sample. In this instance, the composition of the mixed solution used in the gradient elution may be subject to change depending on the isocratic elution conditions.

Additionally, in a state that the first pump connection port 116 and the first outlet port 117 are connected, the first pump P1 feeds the first solvent into the column selection valve 130. Additionally, in a state that the first inlet port 131 and the second column valve connection port 133 are connected, the first solvent fed into the column selection valve 130 is fed into the second column valve 170. Additionally, in a state that the second solid phase extraction column inlet port 171 and the second solid phase extraction column transit port 173 are connected and the second solid phase extraction column connection port 172 and the second sample separation column connection port 174 are connected, the first solvent fed into the second column valve 170 is injected onto the second solid phase extraction column SPE2 and the second sample separation column COL2 to continuously equilibrate the second solid phase extraction column SPE2 and the second sample separation column COL2.

Additionally, in a state that the first sample storage loop connection port 114 and the second sample storage loop connection port 115 are connected to the sample inlet port 112 and the sample outlet port 113, respectively, the sample is fed from the sample injector S and injected and stored in the sample storage loop 111.

Additionally, during separation and analysis of the sample in the first sample separation column COL1, the first pump P1 injects the first solvent onto the sample storage loop 111 to inject the sample stored in the sample storage loop 111 onto the second solid phase extraction column SPE2 and desalt and concentrate the sample.

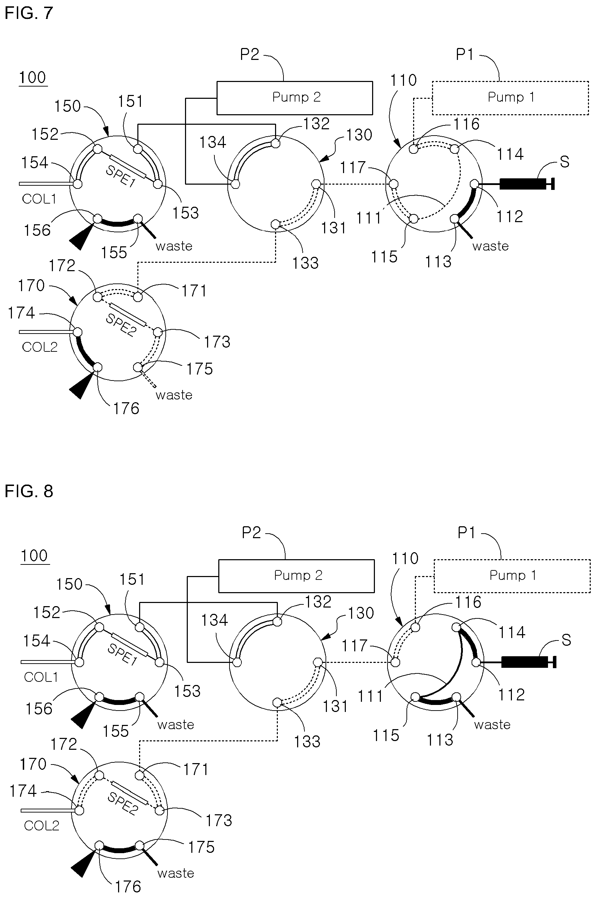

That is, as shown in FIG. 7, in a state that the second solid phase extraction column inlet port 171 and the second solid phase extraction column connection port 172 are connected and the second solid phase extraction column transit port 173 and the third outlet port 175 are connected, the sample stored in the sample storage loop 111 is injected onto the second solid phase extraction column SPE2 together with the first solvent fed from the first pump P1, and is desalted and concentrated. Specifically, in a state that the first pump connection port 116 and the first sample storage loop connection port 114 are connected and the second sample storage loop connection port 115 and the first outlet port 117 are connected, the sample stored in the sample storage loop 111 is fed into the column selection valve 130 together with the first solvent fed from the first pump P1. Additionally, in a state that the first inlet port 131 and the second column valve connection port 133 are connected, the first solvent and the sample fed into the column selection valve 130 is fed into the second column valve 170. Additionally, in a state that the second solid phase extraction column inlet port 171 and the second solid phase extraction column connection port 172 are connected and the second solid phase extraction column transit port 173 and the third outlet port 175 are connected, the first solvent and the sample fed into the second column valve 170 is injected onto the second solid phase extraction column SPE2. Additionally, the sample is desalted and concentrated in the second solid phase extraction column SPE2 by additionally injecting the first solvent, for example, in an amount of about 2 µL to cause salts to melt in the first solvent which is a water-soluble solvent and exit the third outlet port 175.

Additionally, during separation analysis of the sample in the first sample separation column COL1, the first pump P1 injects the mixed solution containing the first solvent and the second solvent onto the second solid phase extraction column SPE2 and the second sample separation column COL2 to perform isocratic elution.

Specifically, as shown in FIG. 8, in a state that the first pump connection port 116 and the first outlet port 117 are connected, the first pump P1 feeds the mixed solution containing the first solvent and the second solvent into the column selection valve 130. Additionally, in a state that the first inlet port 131 and the second column valve connection port 133 are connected, the mixed solution containing the first solvent and the second solvent fed into the column selection valve 130 is fed into the second column valve 170. Additionally, in a state that the second solid phase extraction column inlet port 171 and the second solid phase extraction column transit port 173 are connected and the second solid phase extraction column connection port 172 and the second sample separation column connection port 174 are connected, the mixed solution containing the first solvent and the second solvent fed into the second column valve 170 is injected onto the second solid phase extraction column SPE2 and the second sample separation column COL2 to perform isocratic elution.

Additionally, after the third mode, referring to FIG. 8, the method for controlling the mutually independent dual online liquid chromatography device 100 carries out a fourth mode.

In the fourth mode, separation and analysis of the sample in the first sample separation column COL1 and isocratic elution from the second solid phase extraction column SPE2 and the second sample separation column COL2 are completed at the same time.

The end time of separation and analysis of the sample in the first sample separation column COL1 and the time immediately before the first peptide is eluted from the second sample separation column COL2 are matched to each other so that separation and analysis in the second sample separation column COL2 starts as soon as separation and analysis in the first sample separation column COL1 ends, thereby minimizing the mass signal dead time of the mass spectrometer (MS).

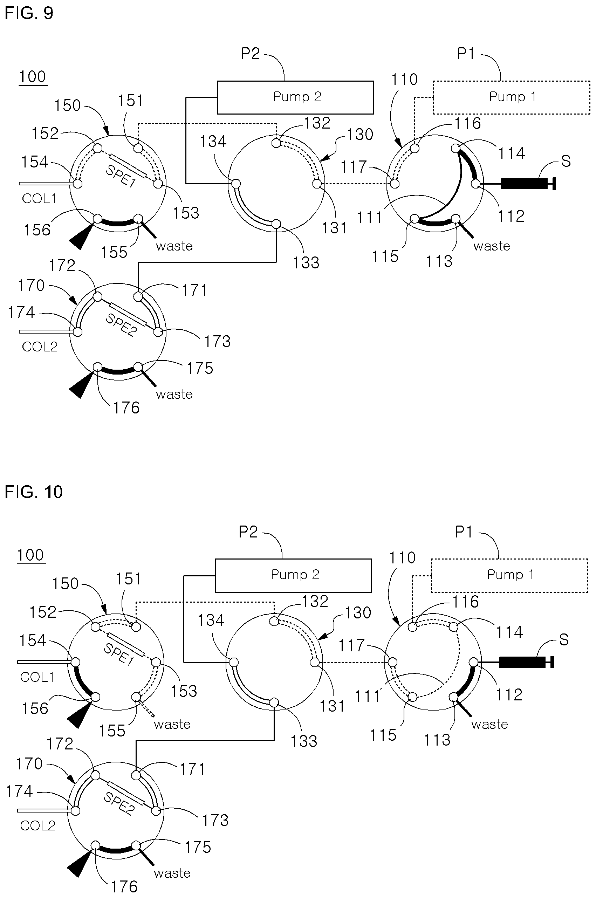

Additionally, after the fourth mode, referring to FIG. 9, the method for controlling the mutually independent dual online liquid chromatography device 100 carries out a fifth mode.

In the fifth mode, during gradient elution from the second solid phase extraction column SPE2 and the second sample separation column COL2 and separation and analysis of the sample, wash and equilibration, sample injection and isocratic elution of the first solid phase extraction column SPE1 and the first sample separation column COL1 are performed.

That is, when the isocratic elution from the second solid phase extraction column SPE2 and the second sample separation column COL2 is completed, the second pump P2 feeds the mixed solution containing the increasing second solvent over time into the second solid phase extraction column SPE2 and the second sample separation column COL2 to perform gradient elution and separation and analysis of the sample, and at the same time, the first pump P1 feeds the first solvent into the first solid phase extraction column SPE1 and the first sample separation column COL1 to continuously equilibrate the first solid phase extraction column SPE1 and the first sample separation column COL1, and store the sample in the sample storage loop 111. On the other hand, before the equilibration of the first solid phase extraction column SPE1 and the first sample separation column COL1, the washing process of the first solid phase extraction column SPE1 and the first sample separation column COL1 may be performed for a short time using the mixed solution containing 20% the first solvent and 80% the second solvent, and the washing process of the first solid phase extraction column SPE1 and the first sample separation column COL1 may be performed repeatedly multiple times (see FIG. 1).

Specifically, as shown in FIG. 9, in a state that the second pump connection port 134 and the second column valve connection port 133 are connected, the second pump P2 feeds the mixed solution containing the first solvent and the second solvent into the second column valve 170. Additionally, in a state that the second solid phase extraction column inlet port 171 and the second solid phase extraction column transit port 173 are connected and the second solid phase extraction column connection port 172 and the second sample separation column connection port 174 are connected, the mixed solution fed into the second column valve 170 is injected onto the second solid phase extraction column SPE2 and the second sample separation column COL2 to perform gradient elution and separation and analysis of the sample. As shown in FIG. 1, the gradient elution is performed with varying ratios of the first solvent and the second solvent in the mixed solution containing the first solvent and the second solvent over time using the second pump P2. That is, the initial condition of the mixed solution starts from 90% the first solvent and 10% the second solvent in the same way as the isocratic elution condition, and as the dissociation level of the sample increases with the increasing proportion of the second solvent in the mixed solution, the sample injected onto the second solid phase extraction column SPE2 is introduced into the second sample separation column COL2, and subsequently, analysis is conducted through separation of the sample. In this instance, the composition of the mixed solution used in the gradient elution may be subject to change depending on the isocratic elution conditions.

Additionally, in a state that the first pump connection port 116 and the first outlet port 117 are connected, the first pump P1 feeds the first solvent into the column selection valve 130. Additionally, in a state that the first inlet port 131 and the first column valve connection port 132 are connected, the first solvent fed into the column selection valve 130 is fed into the first column valve 150. Additionally, in a state that the first solid phase extraction column inlet port 151 and the first solid phase extraction column transit port 153 are connected and the first solid phase extraction column connection port 152 and the first sample separation column connection port 154 are connected, the first solvent fed into the first column valve 150 is injected onto the first solid phase extraction column SPE1 and the first sample separation column COL1 to continuously equilibrate the first solid phase extraction column SPE1 and the first sample separation column COL1.

Additionally, in a state that the first sample storage loop connection port 114 and the second sample storage loop connection port 115 are connected to the sample inlet port 112 and the sample outlet port 113, respectively, the sample is fed from the sample injector S and injected and stored in the sample storage loop 111.

Additionally, during separation and analysis of the sample in the second sample separation column COL2, the first pump P1 feeds the first solvent into the sample storage loop 111 to inject the sample stored in the sample storage loop 111 onto the first solid phase extraction column and desalt and concentrate the sample.

That is, as shown in FIG. 10, in a state that the first solid phase extraction column inlet port 151 and the first solid phase extraction column connection port 152 are connected and the first solid phase extraction column transit port 153 and the second outlet port 155 are connected, the sample stored in the sample storage loop 111 is injected onto the first solid phase extraction column SPE1 together with the first solvent fed from the first pump P1, and is desalted and concentrated. Specifically, in a state that the first pump connection port 116 and the first sample storage loop connection port 114 are connected and the second sample storage loop connection port 115 and the first outlet port 117 are connected, the sample stored in the sample storage loop 111 is fed into the column selection valve 130 together with the first solvent fed from the first pump P1. Additionally, in a state that the first inlet port 131 and the first column valve connection port 132 are connected, the first solvent and the sample fed into the column selection valve 130 are fed into the first column valve 150. Additionally, in a state that the first solid phase extraction column inlet port 151 and the first solid phase extraction column connection port 152 are connected and the first solid phase extraction column transit port 153 and the second outlet port 155 are connected, the first solvent and the sample fed into the first column valve 150 are injected onto the first solid phase extraction column SPE1. Additionally, the sample is desalted and concentrated in the first solid phase extraction column SPE1 by additionally injecting the first solvent, for example, in an amount of about 2 µL to cause salts to melt in the first solvent which is a water-soluble solvent and exit the second outlet port 155.

Additionally, during separation and analysis of the sample in the second sample separation column COL2, the first pump P1 injects the mixed solution containing the first solvent and the second solvent onto the first solid phase extraction column SPE1 and the first sample separation column COL1 to perform isocratic elution.

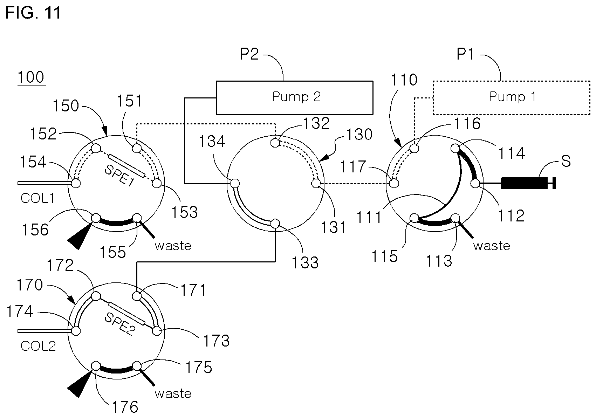

Specifically, as shown in FIG. 11, in a state that the first pump connection port 116 and the first outlet port 117 are connected, the first pump P1 feeds the mixed solution containing the first solvent and the second solvent into the column selection valve 130. Additionally, in a state that the first inlet port 131 and the first column valve connection port 132 are connected, the mixed solution containing the first solvent and the second solvent fed into the column selection valve 130 is fed into the first column valve 150. Additionally, in a state that the first solid phase extraction column inlet port 151 and the first solid phase extraction column transit port 153 are connected and the first solid phase extraction column connection port 152 and the first sample separation column connection port 154 are connected, the mixed solution containing the first solvent and the second solvent fed into the first column valve 150 is injected onto the first solid phase extraction column SPE1 and the first sample separation column COL1 to perform isocratic elution.

Additionally, after the fifth mode, referring to FIG. 11, the method for controlling the mutually independent dual online liquid chromatography device 100 carries out a sixth mode.

In the sixth mode, separation and analysis of the sample in the second sample separation column COL2 and isocratic elution from the first solid phase extraction column SPE1 and the first sample separation column COL1 are completed at the same time.

The end time of separation analysis of the sample in the second sample separation column COL2 and the time immediately before the first peptide is eluted from the first sample separation column COL1 are matched to each other so that separation and analysis in the first sample separation column COL1 starts as soon as separation and analysis in the second sample separation column COL2 ends, thereby minimizing the mass signal dead time of the mass spectrometer (MS).

Additionally, after the sixth mode, the method for controlling the mutually independent dual online liquid chromatography device 100 may carry out the third to sixth modes in a sequential order repeatedly according to the number of times of separation and analysis of the sample or the number of samples.

In this instance, in the third mode, before the equilibration of the second solid phase extraction column SPE2 and the second sample separation column COL2, the washing process of the second solid phase extraction column SPE2 and the second sample separation column COL2 may be additionally performed for a short time using the mixed solution containing 20% the first solvent and 80% the second solvent, and the washing process of the second solid phase extraction column SPE2 and the second sample separation column COL2 may be performed repeatedly multiple times (see FIGS. 1 and 6).

As described above, since the method for controlling the mutually independent dual online liquid chromatography device 100 may iteratively carry out the third to sixth modes according to the number of times of separation and analysis of the sample or the number of samples, it is possible to improve the efficiency of separation and analysis without the influence of the dead time.

Additionally, since the method for controlling the mutually independent dual online liquid chromatography device 100 according to the present disclosure adds the isocratic elution process, it is possible to minimize the mass signal dead time during which peptide is not detected in the mass spectrometer (MS) and improve the separation performance of hydrophilic peptides.

The present disclosure is not limited to the disclosed embodiments, and it is obvious to those skilled in the art that various modifications and changes may be made thereto without departing from the scope of the present disclosure.

### [Detailed Description of Main Elements]

- 100:: Dual online liquid chromatography device
- 110:: Sample inlet valve
- 111:: Sample storage loop
- 112:: Sample inlet port
- 113:: Sample outlet port
- 114:: First sample storage loop connection port
- 115:: Second sample storage loop connection port
- 116:: First pump connection port
- 117:: First outlet port
- 130:: Column selection valve
- 131:: First inlet port
- 132:: First column valve connection port
- 133:: Second column valve connection port
- 134:: Second pump connection port
- 150:: First column valve
- COL1:: First sample separation column
- SPE1:: First solid phase extraction column
- 151:: First solid phase extraction column inlet port
- 152:: First solid phase extraction column connection port
- 153:: First solid phase extraction column transit port
- 154:: First sample separation column connection port
- 155:: Second outlet port
- 156:: First column outlet port
- 170:: Second column valve
- COL2:: Second sample separation column
- SPE2:: Second solid phase extraction column
- 171:: Second solid phase extraction column inlet port
- 172:: Second solid phase extraction column connection port
- 173:: Second solid phase extraction column transit port
- 174:: Second sample separation column connection port
- 175:: Third outlet port
- 176:: Second column outlet port
- P1:: First pump
- P2:: Second pump
- S:: Sample injector

## Claims

1. A method for controlling a mutually independent dual online liquid chromatography device, comprising:
(A) injecting, by a sample injector (S) connected to a sample inlet valve (110), a sample onto a sample storage loop (111) of the sample inlet valve (110) to store the sample, and at the same time, injecting, by a first pump (P1) connected to the sample inlet valve (110), a first solvent onto a first solid phase extraction column (SPE1) and a first sample separation column (COL1) of a first column valve (150) via the sample inlet valve (110) and a column selection valve (130) to equilibrate the first solid phase extraction column (SPE1) and the first sample separation column (COL1), and injecting, by a second pump (P2) connected to the column selection valve (130), the first solvent onto a second solid phase extraction column (SPE2) and a second sample separation column (COL2) of a second column valve (170) via the column selection valve (130) to equilibrate the second solid phase extraction column (SPE2) and the second sample separation column (COL2);
(B) performing valve switching on the sample inlet valve (110) and the first column valve (150) to inject the sample stored in the sample storage loop (111) onto the first solid phase extraction column (SPE1) via the column selection valve (130) together with the first solvent fed from the first pump (P1), and desalting and concentrating the sample, and at the same time, injecting, by the second pump (P2), the first solvent onto the second solid phase extraction column (SPE2) and the second sample separation column (COL2) via the column selection valve (130) to continuously equilibrate the second solid phase extraction column (SPE2) and the second sample separation column (COL2);
(C) performing valve switching on the sample inlet valve (110) and the first column valve (150) again, injecting, by the first pump (P1), a mixed solution of the first solvent and a second solvent onto the first solid phase extraction column (SPE1) and the first sample separation column (COL1) via the sample inlet valve (110) and the column selection valve (130) to perform isocratic elution[fig.5][70], and at the same time, injecting, by the second pump (P2), the first solvent onto the second solid phase extraction column (SPE2) and the second sample separation column (COL2) via the column selection valve (130) to continuously equilibrate the second solid phase extraction column (SPE2) and the second sample separation column (COL2);
(D) performing valve switching of the column selection valve (130) to inject, by the second pump (P2), the mixed solution containing the increasing second solvent over time onto the first solid phase extraction column (SPE1) and the first sample separation column (COL1) via the column selection valve (130) to perform gradient elution for separation and analysis of the sample, and at the same time, injecting, by the first pump (P1), the first solvent onto the second solid phase extraction column (SPE2) and the second sample separation column (COL2) via the sample inlet valve (110) and the column selection valve (130) to continuously equilibrate the second solid phase extraction column (SPE2) and the second sample separation column (COL2);
(E) during the separation and analysis of the sample by the gradient elution in the first sample separation column (COL1), injecting, by the sample injector (S), the sample onto the sample storage loop (111) to store the sample, and at the same time, injecting, by the first pump (P1) connected to the sample inlet valve (110), the first solvent onto the second solid phase extraction column (SPE2) and the second sample separation column (COL2) of the second column valve (170) via the sample inlet valve (110) and the column selection valve (130) to continuously equilibrate the second solid phase extraction column (SPE2) and the second sample separation column (COL2);
(F) during the separation and analysis of the sample by the gradient elution in the first sample separation column (COL1), performing valve switching on the sample inlet valve (110) and the second column valve (170), injecting the sample stored in the sample storage loop (111) onto the second solid phase extraction column (SPE2) via the sample inlet valve (110) and the column selection valve (130) together with the first solvent fed from the first pump (P1), and desalting and concentrating the sample;
(G) during the separation and analysis of the sample by the gradient elution in the first sample separation column (COL1), performing valve switching on the sample inlet valve (110) and the second column valve (170) and injecting, by the first pump (P1), the mixed solution of the first solvent and the second solvent onto the second solid phase extraction column (SPE2) and the second sample separation column (COL2) via the sample inlet valve (110) and the column selection valve (130) to perform isocratic elution;
(H) completing the separation and analysis of the sample in the first sample separation column (COL1) and the isocratic elution from the second solid phase extraction column (SPE2) and the second sample separation column (COL2) at the same time;
(I) performing valve switching of the column selection valve (130) to inject, by the second pump (P2), the mixed solution containing the increasing second solvent over time onto the second solid phase extraction column (SPE2) and the second sample separation column (COL2) via the column selection valve (130) to perform gradient elution for separation and analysis of the sample, and at the same time, injecting, by the first pump (P1), the mixed solution containing the first solvent and the second solvent onto the first solid phase extraction column (SPE1) and the first sample separation column (COL1) via the sample inlet valve (110) and the column selection valve (130) to wash the first solid phase extraction column (SPE1) and the first sample separation column (COL1);
(J) during the separation and analysis of the sample by the gradient elution in the second sample separation column (COL2), injecting, by the first pump (P1), the first solvent onto the first solid phase extraction column (SPE1) and the first sample separation column (COL1) via the sample inlet valve (110) and the column selection valve (130) to equilibrate the first solid phase extraction column (SPE1) and the first sample separation column (COL1);
(K) during the separation and analysis of the sample by the gradient elution in the second sample separation column (COL2), injecting, by the sample injector (S), the sample onto the sample storage loop (111) to store the sample, and at the same time, injecting, by the first pump (P1) connected to the sample inlet valve (110), the first solvent onto the first solid phase extraction column (SPE1) and the first sample separation column (COL1) of the first column valve (150) via the sample inlet valve (110) and the column selection valve (130) to continuously equilibrate the first solid phase extraction column (SPE1) and the first sample separation column (COL1);
(L) during the separation and analysis of the sample by the gradient elution in the second sample separation column (COL2), performing valve switching on the sample inlet valve (110) and the first column valve (150) to inject the sample stored in the sample storage loop (111) onto the first solid phase extraction column (SPE1) via the sample inlet valve (110) and the column selection valve (130) together with the first solvent fed from the first pump (P1), and desalting and concentrating the sample;
(M) during the separation and analysis of the sample by the gradient elution in the second sample separation column (COL2), performing valve switching on the sample inlet valve (110) and the first column valve (150), and injecting, by the first pump (P1), the mixed solution of the first solvent and the second solvent onto the first solid phase extraction column (SPE1) and the first sample separation column (COL1) via the sample inlet valve (110) and the column selection valve (130) to perform isocratic elution;
(N) completing the separation and analysis of the sample in the second sample separation column (COL2) and the isocratic elution from the first solid phase extraction column (SPE1) and the first sample separation column (COL1) at the same time ; and
(O) performing the steps (D) to (N) in a sequential order repeatedly according to the number of times of separation and analysis of the sample or the number of samples.

2. The method for controlling a mutually independent dual online liquid chromatography device according to claim 1, wherein the sample inlet valve (110) includes:
a sample inlet port (112) connected to the sample injector (S);
a sample outlet port (113) disposed adjacent to the sample inlet port (112);
a first pump connection port (116) connected to the first pump (P1);
a first outlet port (117) disposed adjacent to the first pump connection port (116) and connected to the column selection valve (130); and
a first sample storage loop connection port (114) and a second sample storage loop connection port (115) to which two ends of the sample storage loop (111) are connected respectively,
wherein the column selection valve (130) includes:
a first inlet port (131) connected to the first outlet port (117);
a first column valve connection port (132) connected to the first column valve (150);
a second column valve connection port (133) connected to the second column valve (170); and
a second pump connection port (134) connected to the second pump (P2), and
wherein the step (A) comprises,
in a state that the first sample storage loop connection port (114) and the second sample storage loop connection port (115) are connected to the sample inlet port (112) and the sample outlet port (113), respectively, receiving the sample from the sample injector (S) and injecting and storing the sample in the sample storage loop (111),
in a state that the first pump connection port (116), the first outlet port (117), the first inlet port (131) and the first column valve connection port (132) are connected, feeding, by the first pump (P1), the first solvent into the column selection valve (130), feeding the first solvent fed into the column selection valve (130) into the first column valve (150) and injecting onto the first solid phase extraction column (SPE1) and the first sample separation column (COL1) to equilibrate the first solid phase extraction column (SPE1) and the first sample separation column (COL1), and
in a state that the second pump connection port (134) and the second column valve connection port (133) are connected, feeding, by the second pump (P2), the first solvent into the column selection valve (130), feeding the first solvent fed into the column selection valve (130) into the second column valve (170) and injecting onto the second solid phase extraction column (SPE2) and the second sample separation column (COL2) to equilibrate the second solid phase extraction column (SPE2) and the second sample separation column (COL2).

3. The method for controlling a mutually independent dual online liquid chromatography device according to claim 2, wherein the first column valve (150) includes:
a first solid phase extraction column connection port (152) and a first solid phase extraction column transit port (153) connected to two ends of the first solid phase extraction column (SPE1), respectively;
a first solid phase extraction column inlet port (151) connected to the first column valve connection port (132), and selectively connected to the first solid phase extraction column connection port (152) and the first solid phase extraction column transit port (153);
a first sample separation column connection port (154) connected to the first sample separation column (COL1), and connected to or disconnected from the first solid phase extraction column connection port (152);
a second outlet port (155) disposed adjacent to the first solid phase extraction column transit port (153) and connected to or disconnected from the first solid phase extraction column transit port (153); and
a first column outlet port (156) disposed adjacent to the first sample separation column connection port (154) and selectively connected to the first sample separation column connection port (154) and the second outlet port (155),
wherein the second column valve (170) includes:
a second solid phase extraction column connection port (172) and a second solid phase extraction column transit port (173) connected to two ends of the second solid phase extraction column (SPE2), respectively;
a second solid phase extraction column inlet port (171) connected to the second column valve connection port (133), and selectively connected to the second solid phase extraction column connection port (172) and the second solid phase extraction column transit port (173);
a second sample separation column connection port (174) connected to the second sample separation column (COL2), and connected to or disconnected from the second solid phase extraction column connection port (172);
a third outlet port (175) disposed adjacent to the second solid phase extraction column transit port (173) and connected to or disconnected from the second solid phase extraction column transit port (173); and
a second column outlet port (176) disposed adjacent to the second sample separation column connection port (174) and selectively connected to the second sample separation column connection port (174) and the third outlet port (175),
wherein the step (B) comprises,
in a state that the first pump connection port (116), the first sample storage loop connection port (114), the second sample storage loop connection port (115) and the first outlet port (117) are connected, the first inlet port (131) and the first column valve connection port (132) are connected, the first solid phase extraction column inlet port (151) and the first solid phase extraction column connection port (152) are connected and the first solid phase extraction column transit port (153) and the second outlet port (155) are connected, injecting the sample stored in the sample storage loop (111) onto the first solid phase extraction column (SPE1) together with the first solvent fed from the first pump (P1) and desalting and concentrating the sample, and
in a state that the second pump connection port (134) and the second column valve connection port (133) are connected, the second solid phase extraction column inlet port (171) and the second solid phase extraction column transit port (173) are connected and the second solid phase extraction column connection port (172) and the second sample separation column connection port (174) are connected, injecting, by the second pump (P2), the first solvent onto the second solid phase extraction column (SPE2) and the second sample separation column (COL2) to continuously equilibrate the second solid phase extraction column (SPE2) and the second sample separation column (COL2).

4. The method for controlling a mutually independent dual online liquid chromatography device according to claim 3, wherein the step (C) comprises,
in a state that the first pump connection port (116) and the first outlet port (117) are connected, the first inlet port (131) and the first column valve connection port (132) are connected, the first solid phase extraction column inlet port (151) and the first solid phase extraction column transit port (153) are connected and the first solid phase extraction column connection port (152) and the first sample separation column connection port (154) are connected, injecting, by the first pump (P1), the mixed solution containing the first solvent and the second solvent onto the first solid phase extraction column (SPE1) and the first sample separation column (COL1) to perform the isocratic elution, and
in a state that the second pump connection port (134) and the second column valve connection port (133) are connected, the second solid phase extraction column inlet port (171) and the second solid phase extraction column transit port (173) are connected, and the second solid phase extraction column connection port (172) and the second sample separation column connection port (174) are connected, injecting, by the second pump (P2), the first solvent onto the second solid phase extraction column (SPE2) and the second sample separation column (COL2) to continuously equilibrate the second solid phase extraction column (SPE2) and the second sample separation column (COL2).

5. The method for controlling a mutually independent dual online liquid chromatography device according to claim 4, wherein the step (D) comprises,
in a state that the second pump connection port (134) and the first column valve connection port (132) are connected, the first solid phase extraction column inlet port (151) and the first solid phase extraction column transit port (153) are connected and the first solid phase extraction column connection port (152) and the first sample separation column connection port (154) are connected, injecting, by the second pump (P2), the mixed solution containing the increasing second solvent over time onto the first solid phase extraction column (SPE1) and the first sample separation column (COL1) to perform the gradient elution for separation and analysis of the sample, and
at the same time, in a state that the first pump connection port (116) and the first outlet port (117) are connected, the first inlet port (131) and the second column valve connection port (133) are connected, the second solid phase extraction column inlet port (171) and the second solid phase extraction column transit port (173) are connected and the second solid phase extraction column connection port (172) and the second sample separation column connection port (174) are connected, injecting, by the first pump (P1), the first solvent onto the second solid phase extraction column (SPE2) and the second sample separation column (COL2) to continuously equilibrate the second solid phase extraction column (SPE2) and the second sample separation column (COL2),
wherein the step (E) comprises,
in a state that the second pump connection port (134) and the first column valve connection port (132) are connected, the first solid phase extraction column inlet port (151) and the first solid phase extraction column transit port (153) are connected and the first solid phase extraction column connection port (152) and the first sample separation column connection port (154) are connected, injecting, by the second pump (P2), the mixed solution containing the increasing second solvent over time onto the first solid phase extraction column (SPE1) and the first sample separation column (COL1) to perform gradient elution for separation and analysis of the sample,
at the same time, in a state that the first sample storage loop connection port (114) and the second sample storage loop connection port (115) are connected to the sample inlet port (112) and the sample outlet port (113), respectively, receiving the sample from the sample injector (S) and injecting and storing the sample in the sample storage loop (111), and
at the same time, in a state that the first pump connection port (116) and the first outlet port (117) are connected, the first inlet port (131) and the second column valve connection port (133) are connected, the second solid phase extraction column inlet port (171) and the second solid phase extraction column transit port (173) are connected and the second solid phase extraction column connection port (172) and the second sample separation column connection port (174) are connected, injecting, by the first pump (P1), the first solvent onto the second solid phase extraction column (SPE2) and the second sample separation column (COL2) to continuously equilibrate the second solid phase extraction column (SPE2) and the second sample separation column (COL2),
wherein the step (F) comprises,
in a state that the second pump connection port (134) and the first column valve connection port (132) are connected, the first solid phase extraction column inlet port (151) and the first solid phase extraction column transit port (153) are connected and the first solid phase extraction column connection port (152) and the first sample separation column connection port (154) are connected, feeding, by the second pump (P2), the mixed solution containing the increasing second solvent over time onto the first solid phase extraction column (SPE1) and the first sample separation column (COL1) to perform gradient elution for separation and analysis of the sample[81][78], and at the same time, in a state that the first pump connection port (116), the first sample storage loop connection port (114), the second sample storage loop connection port (115) and the first outlet port (117) are connected, the first inlet port (131) and the second column valve connection port (133) are connected, the second solid phase extraction column inlet port (171) and the second solid phase extraction column connection port (172) are connected and the second solid phase extraction column transit port (173) and the third outlet port (175) are connected, injecting the sample stored in the sample storage loop (111) onto the second solid phase extraction column (SPE2) together with the first solvent fed from the first pump (P1) and desalting and concentrating the sample, and
wherein the step (G) comprises,
in a state that the second pump connection port (134) and the first column valve connection port (132) are connected, the first solid phase extraction column inlet port (151) and the first solid phase extraction column transit port (153) are connected and the first solid phase extraction column connection port (152) and the first sample separation column connection port (154) are connected, injecting, by the second pump (P2), the mixed solution containing the increasing second solvent over time onto the first solid phase extraction column (SPE1) and the first sample separation column (COL1) to perform the gradient elution for separation and analysis of the sample[83][78], and at the same time, in a state that the first pump connection port (116) and the first outlet port (117) are connected, the first inlet port (131) and the second column valve connection port (133) are connected, the second solid phase extraction column inlet port (171) and the second solid phase extraction column transit port (173) are connected and the second solid phase extraction column connection port (172) and the second sample separation column connection port (174) are connected, injecting, by the first pump (P1), the mixed solution containing the first solvent and the second solvent onto the second solid phase extraction column (SPE2) and the second sample separation column (COL2) to perform the isocratic elution.

6. The method for controlling a mutually independent dual online liquid chromatography device according to claim 5, wherein when the step (I) comprises,
in a state that the second pump connection port (134) and the second column valve connection port (133) are connected, the second solid phase extraction column inlet port (171) and the second solid phase extraction column transit port (173) are connected and the second solid phase extraction column connection port (172) and the second sample separation column connection port (174) are connected, feeding, by the second pump (P2), the mixed solution containing the increasing second solvent over time into the second solid phase extraction column (SPE2) and the second sample separation column (COL2) to perform the gradient elution for separation and analysis of the sample, and
at the same time, in a state that the first pump connection port (116) and the first outlet port (117) are connected, the first inlet port (131) and the first column valve connection port (132) are connected, the first solid phase extraction column inlet port (151) and the first solid phase extraction column transit port (153) are connected and the first solid phase extraction column connection port (152) and the first sample separation column connection port (154) are connected, injecting, by the first pump (P1), the mixed solution containing the first solvent and the second solvent onto the first solid phase extraction column (SPE1) and the first sample separation column (COL1) to wash the first solid phase extraction column (SPE1) and the first sample separation column (COL1),
wherein the step (J) comprises,
in a state that the second pump connection port (134) and the second column valve connection port (133) are connected, the second solid phase extraction column inlet port (171) and the second solid phase extraction column transit port (173) are connected and the second solid phase extraction column connection port (172) and the second sample separation column connection port (174) are connected, feeding, by the second pump (P2), the mixed solution containing the increasing second solvent over time into the second solid phase extraction column (SPE2) and the second sample separation column (COL2) to perform the gradient elution for separation and analysis of the sample, and
at the same time, in a state that the first pump connection port (116) and the first outlet port (117) are connected, the first inlet port (131) and the first column valve connection port (132) are connected, the first solid phase extraction column inlet port (151) and the first solid phase extraction column transit port (153) are connected and the first solid phase extraction column connection port (152) and the first sample separation column connection port (154) are connected, injecting, by the first pump (P1), the first solvent onto the first solid phase extraction column (SPE1) and the first sample separation column (COL1) to equilibrate the first solid phase extraction column (SPE1) and the first sample separation column (COL1),
wherein the step (K) comprises,
in a state that the second pump connection port (134) and the second column valve connection port (133) are connected, the second solid phase extraction column inlet port (171) and the second solid phase extraction column transit port (173) are connected and the second solid phase extraction column connection port (172) and the second sample separation column connection port (174) are connected, injecting, by the second pump (P2), the mixed solution containing the increasing second solvent over time onto the second solid phase extraction column (SPE2) and the second sample separation column (COL2) to perform the gradient elution for separation and analysis of the sample,
at the same time, in a state that the first sample storage loop connection port (114) and the second sample storage loop connection port (115) are connected to the sample inlet port (112) and the sample outlet port (113), respectively, receiving the sample from the sample injector (S) and injecting and storing the sample in the sample storage loop (111), and
at the same time, in a state that the first pump connection port (116) and the first outlet port (117) are connected, the first inlet port (131) and the first column valve connection port (132) are connected, the first solid phase extraction column inlet port (151) and the first solid phase extraction column transit port (153) are connected and the first solid phase extraction column connection port (152) and the first sample separation column connection port (154) are connected, injecting, by the first pump (P1), the first solvent onto the first solid phase extraction column (SPE1) and the first sample separation column (COL1) to continuously equilibrate the first solid phase extraction column (SPE1) and the first sample separation column (COL1),
wherein the step (L) comprises,
in a state that the second pump connection port (134) and the second column valve connection port (133) are connected, the second solid phase extraction column inlet port (171) and the second solid phase extraction column transit port (173) are connected and the second solid phase extraction column connection port (172) and the second sample separation column connection port (174) are connected, injecting, by the second pump (P2), the mixed solution containing the increasing second solvent over time onto the second solid phase extraction column (SPE2) and the second sample separation column (COL2) to perform the gradient elution for separation and analysis of the sample, and
at the same time, in a state that the first pump connection port (116), the first sample storage loop connection port (114), the second sample storage loop connection port (115) and the first outlet port (117) are connected, the first inlet port (131) and the first column valve connection port (132) are connected, the first solid phase extraction column inlet port (151) and the first solid phase extraction column connection port (152) are connected and the first solid phase extraction column transit port (153) and the second outlet port (155) are connected, injecting the sample stored in the sample storage loop onto the first solid phase extraction column together with the first solvent fed from the first pump and desalting and concentrating the sample, and
wherein the step (M) comprises,
in a state that the second pump connection port (134) and the second column valve connection port (133) are connected, the second solid phase extraction column inlet port (171) and the second solid phase extraction column transit port (173) are connected and the second solid phase extraction column connection port (172) and the second sample separation column connection port (174) are connected, injecting, by the second pump (P2), the mixed solution containing the increasing second solvent over time onto the second solid phase extraction column (SPE2) and the second sample separation column (COL2) to perform the gradient elution for separation and analysis of the sample, and
at the same time, in a state that the first pump connection port (116) and the first outlet port (117) are connected, the first inlet port (131) and the first column valve connection port (132) are connected, the first solid phase extraction column inlet port (151) and the first solid phase extraction column transit port (153) are connected and the first solid phase extraction column connection port (152) and the first sample separation column connection port (154) are connected, feeding, by the first pump (P1), the mixed solution containing the first solvent and the second solvent onto the first solid phase extraction column (SPE1) and the first sample separation column (COL1) to perform the isocratic elution.

7. The method for controlling a mutually independent dual online liquid chromatography device according to claim 5, wherein when the step (O) comprises repeatedly performing the steps (D) to (N) in a sequential order, the step (D) comprises, before equilibrating the second solid phase extraction column (SPE2) and the second sample separation column (COL2), in a state that the first pump connection port (116) and the first outlet port (117) are connected and the first inlet port (131) and the second column valve connection port (133) are connected, injecting, by the first pump (P1), the mixed solution containing the first solvent and the second solvent onto the second solid phase extraction column (SPE2) and the second sample separation column (COL2) to wash the second solid phase extraction column (SPE2) and the second sample separation column (COL2).

8. The method for controlling a mutually independent dual online liquid chromatography device according to claim 1, wherein the step (H) comprises matching an end time of the separation and analysis of the sample in the first sample separation column (COL1) with a time immediately before a first peptide is eluted from the second sample separation column (COL2).

9. The method for controlling a mutually independent dual online liquid chromatography device according to claim 1, wherein the step (N) comprises matching an end time of the separation and analysis of the sample in the second sample separation column (COL2) with a time immediately before a first peptide is eluted from the first sample separation column (COL1).

## Patentansprüche

1. Verfahren zur Steuerung einer voneinander unabhängigen dualen Online-Flüssigkeitschromatographievorrichtung, umfassend:
(A) Injizieren einer Probe durch einen Probeninjektor (S), der mit einem Probeneinlassventil (110) verbunden ist, in eine Probenspeicherschleife (111) des Probeneinlassventils (110), um die Probe zu speichern, und gleichzeitig Injizieren eines ersten Lösungsmittels durch eine erste Pumpe (P1), die mit dem Probeneinlassventil (110) verbunden ist, in eine erste Festphasenextraktionssäule (SPE1) und eine erste Probentrennsäule (COL1) eines ersten Säulenventils (150) über das Probeneinlassventil (110) und ein Säulenauswahlventil (130) um die erste Festphasenextraktionssäule (SPE1) und die erste Probentrennsäule (COL1) zu equilibrieren, und Injizieren des ersten Lösungsmittels, durch eine zweite Pumpe (P2), die mit dem Säulenauswahlventil (130) verbunden ist, in eine zweite Festphasenextraktionssäule (SPE2) und eine zweite Probentrennsäule (COL2) eines zweiten Säulenventils (170) über das Säulenauswahlventil (130), um die zweite Festphasenextraktionssäule (SPE2) und die zweite Probentrennsäule (COL2) zu equilibrieren;
(B) Durchführen einer Ventilumschaltung am Probeneinlassventil (110) und am ersten Säulenventil (150), um die in der Probenspeicherschleife (111) gespeicherten Probe über das Säulenauswahlventil (130) zusammen mit dem ersten Lösungsmittel, das von der ersten Pumpe (P1) zugeführt wird, in die erste Festphasenextraktionssäule (SPE1) zu injizieren, und Entsalzen und Konzentrieren der Probe und gleichzeitig Injizieren des ersten Lösungsmittels durch die zweite Pumpe (P2) in die zweite Festphasenextraktionssäule (SPE2) und die zweite Probentrennsäule (COL2) über das Säulenauswahlventil (130) um die zweite Festphasenextraktionssäule (SPE2) und die zweite Probentrennsäule (COL2) kontinuierlich zu equilibrieren;
(C) Durchführen einer erneuten Ventilumschaltung am Probeneinlassventil (110) und am ersten Säulenventil (150), wobei durch die erste Pumpe (P1) eine gemischte Lösung des ersten Lösungsmittels und eines zweiten Lösungsmittels über das Probeneinlassventil (110) und das Säulenauswahlventil (130) in die erste Festphasenextraktionssäule (SPE1) und die erste Probentrennsäule (COL1) injiziert wird, um isokratische Elution [Abb. 5][70] durchzuführen, und gleichzeitig Injizieren des ersten Lösungsmittels durch die zweite Pumpe (P2), über das Säulenauswahlventil (130) auf die zweite Festphasenextraktionssäule (SPE2) und die zweite Probentrennsäule (COL2), um die zweite Festphasenextraktionssäule (SPE2) und die zweite Probentrennsäule (COL2) kontinuierlich zu equilibrieren;
(D) Durchführen einer Ventilumschaltung des Säulenauswahlventils (130), um durch die zweite Pumpe (P2) die gemischte Lösung, die das zunehmende zweite Lösungsmittel über die Zeit enthält, über das Säulenauswahlventil (130) auf die erste Festphasenextraktionssäule (SPE1) und die erste Probentrennungssäule (COL1) zu injizieren, um Gradienteneluierung zur Separation und Analyse der Probe durchzuführen, und gleichzeitig Injizieren des ersten Lösungsmittels durch die erste Pumpe (P1) auf die zweite Festphasenextraktionssäule (SPE2) und auf die zweite Probentrennsäule (COL2) über das Probeneinlassventil (110) und das Säulenauswahlventil (130) um die zweite Festphasenextraktionssäule (SPE2) und die zweite Probentrennsäule (COL2) kontinuierlich zu equilibrieren;
(E) während der Trennung und Analyse der Probe durch Gradientenelution in der ersten Probentrennsäule (COL1), Injizieren der Probe durch den Probeninjektor (S) in die Probenspeicherschleife (111), um die Probe zu speichern, und gleichzeitig Injizieren des ersten Lösungsmittels durch die erste Pumpe (P1), die mit dem Probeneinlassventil (110) verbunden ist, in die zweite Festphasenextraktionssäule (SPE2) und die zweite Probentrennsäule (COL2) des zweiten Säulenventils (170) über das Probeneinlassventil (110) und das Säulenauswahlventil (130), um die zweite Festphasenextraktionssäule (SPE2) und die zweite Probentrennsäule (COL2) kontinuierlich zu equilibrieren;
(F) während der Trennung und Analyse der Probe durch die Gradientenelution in der ersten Probentrennsäule (COL1), Durchführen einer Ventilumschaltung auf dem Probeneinlassventil (110) und dem zweiten Säulenventil (170), Injizieren der in der Probenspeicherschleife (111) gespeicherten Probe auf die zweite Festphasenextraktionssäule (SPE2) über das Probeneinlassventil (110) und das Säulenauswahlventil (130) zusammen mit dem von der ersten Pumpe (P1) zugeführten ersten Lösungsmittel, und Entsalzen und Konzentrieren der Probe;
(G) während der Trennung und Analyse der Probe durch die Gradientenelution in der ersten Probentrennsäule (COL1), Durchführen einer Ventilumschaltung auf dem Probeneinlassventil (110) und dem zweiten Säulenventil (170) und Injizieren der gemischten Lösung des ersten Lösungsmittels und des zweiten Lösungsmittels durch die erste Pumpe (P1) auf die zweite Festphasenextraktionssäule (SPE2) und die zweite Probentrennsäule (COL2) über das Probeneinlassventil (110) und das Säulenauswahlventil (130), um eine isokratische Elution durchzuführen;
(H) Abschließen der Trennung und Analyse der Probe in der ersten Probentrennsäule (COL1) und der isokratischen Elution von der zweiten Festphasenextraktionssäule (SPE2) und der zweiten Probentrennsäule (COL2) zur gleichen Zeit;
(I) Durchführen einer Ventilumschaltung des Säulenauswahlventils (130), um durch die zweite Pumpe (P2) die gemischte Lösung, die das über die Zeit zunehmende zweite Lösungsmittel enthält, auf die zweite Festphasenextraktionssäule (SPE2) und die zweite Probentrennsäule (COL2) über das Säulenauswahlventil (130) zu injizieren, um Gradientenelution zur Trennung und Analyse der Probe durchzuführen, und gleichzeitig Injizieren der gemischten Lösung, die das erste Lösungsmittel und das zweite Lösungsmittel enthält, durch die erste Pumpe (P1) auf die erste Festphasenextraktionssäule (SPE1) und die erste Probentrennsäule (COL1) über das Probeneinlassventil (110) und das Säulenauswahlventil (130), um die erste Festphasenextraktionssäule (SPE1) und die erste Probentrennsäule (COL1) zu waschen;
(J) während der Trennung und Analyse der Probe durch die Gradientenelution in der zweiten Probentrennsäule (COL2), Injizieren des ersten Lösungsmittels durch die erste Pumpe (P1) auf die erste Festphasenextraktionssäule (SPE1) und die erste Probentrennsäule (COL1) über das Probeneinlassventil (110) und das Säulenauswahlventil (130), um die erste Festphasenextraktionssäule (SPE1) und die erste Probentrennsäule (COL1) zu equilibrieren;
(K) während der Trennung und Analyse der Probe durch die Gradientenelution in der zweiten Probentrennsäule (COL2), Injizieren der Probe in die Probenspeicherschleife (111) durch den Probeninjektor (S), um die Probe zu speichern, und gleichzeitig Injizieren des ersten Lösungsmittels durch die erste Pumpe (P1), die mit dem Probeneinlassventil (110) verbunden ist, in die erste Festphasenextraktionssäule (SPE1) und die erste Probentrennsäule (COL1) des ersten Säulenventils (150) über das Probeneinlassventil (110) und das Säulenauswahlventil (130), um die erste Festphasenextraktionssäule (SPE1) und die erste Probentrennsäule (COL1) kontinuierlich zu equilibrieren;
(L) während der Trennung und Analyse der Probe durch die Gradientenelution in der zweiten Probentrennsäule (COL2), Durchführen einer Ventilumschaltung auf dem Probeneinlassventil (110) und dem ersten Säulenventil (150), um die in der Probenspeicherschleife (111) gespeicherte Probe auf die erste Festphasenextraktionssäule (SPE1) über das Probeneinlassventil (110) und das Säulenauswahlventil (130) zusammen mit dem von der ersten Pumpe (P1) zugeführten ersten Lösungsmittel zu injizieren, und Entsalzen und Konzentrieren der Probe;
(M) während der Trennung und Analyse der Probe durch die Gradientenelution in der zweiten Probentrennsäule (COL2), Durchführen einer Ventilumschaltung auf dem Probeneinlassventil (110) und dem ersten Säulenventil (150) und Injizieren der gemischten Lösung des ersten Lösungsmittels und des zweiten Lösungsmittels durch die erste Pumpe (P1) auf die erste Festphasenextraktionssäule (SPE1) und die erste Probentrennsäule (COL1) über das Probeneinlassventil (110) und das Säulenauswahlventil (130), um eine isokratische Elution durchzuführen;
(N) Abschließen der Trennung und Analyse der Probe in der zweiten Probentrennsäule (COL2) und der isokratischen Elution von der ersten Festphasenextraktionssäule (SPE1) und der ersten Probentrennsäule (COL1) zur gleichen Zeit; und
(O) Durchführen der Schritte (D) bis (N) in einer sequentiellen Reihenfolge, die sich nach der Anzahl der Trennungen und Analysen der Probe oder der Anzahl der Proben richtet.

2. Verfahren zur Steuerung einer voneinander unabhängigen dualen Online-Flüssigkeitschromatographievorrichtung nach Anspruch 1, wobei das Probeneinlassventil (110) einschließt:
einen Probeneinlassanschluss (112), verbunden mit dem Probeninjektor (S);
einen Probenauslassanschluss (113), angeordnet neben dem Probeneinlassanschluss (112);
einen ersten Pumpenverbindungsanschluss (116), verbunden mit der ersten Pumpe (P1);
einen ersten Auslassanschluss (117), angeordnet neben dem ersten Pumpenverbindungsanschluss (116) und verbunden mit dem Säulenauswahlventil (130); und
einen ersten Verbindungsanschluss der Probenspeicherschleife (114) und einen zweiten Verbindungsanschluss der Probenspeicherschleife (115), mit denen jeweils die zwei Enden der Probenspeicherschleife (111) verbunden sind,
wobei das Säulenauswahlventil (130) einschließt:
einen ersten Einlassanschluss (131), verbunden mit dem ersten Auslassanschluss (117);
einen ersten Verbindungsanschluss des Säulenventils (132), verbunden mit dem ersten Säulenventil (150);
einen zweiten Verbindungsanschluss des Säulenventils (133), verbunden mit dem zweiten Säulenventil (170); und
einen zweiten Pumpenverbindungsanschluss (134), verbunden mit der zweiten Pumpe (P2), und wobei der Schritt (A) umfasst:
in einem Zustand, in dem der erste Verbindungsanschluss der Probenspeicherschleife (114) und der zweite Verbindungsanschluss der Probenspeicherschleife(115) mit dem Probeneinlassanschluss (112) bzw. dem Probenauslassanschluss (113) verbunden sind, Empfangen der Probe von dem Probeninjektor (S) und Injizieren und Speichern der Probe in der Probenspeicherschleife (111), in einem Zustand, in dem der erste Pumpenverbindungsanschluss (116), der erste Auslassanschluss (117), der erste Einlassanschluss (131) und der erste Säulenventilverbindungsanschluss (132) verbunden sind, Zuführen des ersten Lösungsmittels durch die erste Pumpe (P1) in das Säulenauswahlventil (130), Zuführen des in das Säulenauswahlventil (130) geleiteten ersten Lösungsmittels in das erste Säulenventil (150) und Injizieren auf die erste Festphasenextraktionssäule (SPE1) und die erste Probentrennsäule (COL1), um die erste Festphasenextraktionssäule (SPE1) und die erste Probentrennsäule (COL1) zu equilibrieren, und
in einem Zustand, in dem der zweite Pumpenverbindungsanschluss (134) und der zweite Säulenventilverbindungsanschluss (133) verbunden sind, Zuführen des ersten Lösungsmittels durch die zweite Pumpe (P2) in das Säulenauswahlventil (130), Zuführen des in das Säulenauswahlventil (130) geleiteten ersten Lösungsmittels in das zweite Säulenventil (170) und Injizieren auf die zweite Festphasenextraktionssäule (SPE2) und die zweite Probentrennsäule (COL2), um die zweite Festphasenextraktionssäule (SPE2) und die zweite Probentrennsäule (COL2) zu equilibrieren.

3. Verfahren zur Steuerung einer voneinander unabhängigen dualen Online-Flüssigkeitschromatographievorrichtung nach Anspruch 2, wobei das erste Säulenventil (150) einschließt:
einen Verbindungsanschluss der ersten Festphasenextraktionssäule (152) und einen Transitanschluss der ersten Festphasenextraktionssäule (153), verbunden jeweils mit den zwei Enden der ersten Festphasenextraktionssäule (SPE1);
einen ersten Einlassanschluss der Festphasenextraktionssäule (151), verbunden mit dem Verbindungsanschluss des ersten Säulenventils (132),und selektiv verbunden mit dem Verbindungsanschluss der ersten Festphasenextraktionssäule (152) und dem Transitanschluss der ersten Festphasenextraktionssäule (153);
einen Verbindungsanschluss der ersten Probentrennsäule (154), verbunden mit der ersten Probentrennsäule (COL1) und verbunden mit oder getrennt von dem Verbindungsanschluss der ersten Festphasenextraktionssäule (152);
einen zweiten Auslassanschluss (155), angeordnet angrenzend an den Transitanschluss der ersten Festphasenextraktionssäule (153) und verbunden mit oder getrennt von dem Transitanschluss der ersten Festphasenextraktionssäule (153); und
einen ersten Säulenauslassanschluss (156), angeordnet angrenzend an den Verbindungsanschluss der ersten Probentrennsäule (154) und selektiv verbunden mit dem Verbindungsanschluss der ersten Probentrennsäule (154) und dem zweiten Auslassanschluss (155)ist,
wobei das zweite Säulenventil (170) einschließt:
einen Verbindungsanschluss der zweiten Festphasenextraktionssäule (172) und einen Transitanschluss der zweiten Festphasenextraktionssäule (173), verbunden jeweils mit den zwei Enden der zweiten Festphasenextraktionssäule (SPE2);
einen Einlassanschluss der zweiten Festphasenextraktionssäule (171), verbunden mit dem zweiten Verbindungsanschluss des Säulenventils (133) und selektiv verbunden mit dem Verbindungsanschluss der zweiten Festphasenextraktionssäule (172) und dem Transitanschluss der zweiten Festphasenextraktionssäule (173);
einen Verbindunganschluss der zweiten Probentrennsäule (174), verbunden mit der zweiten Probentrennsäule (COL2) und verbunden mit oder getrennt von dem Verbindungsanschluss der zweiten Festphasenextraktionssäule (172);
einen dritten Auslassanschluss (175), angeordnet angrenzend an den Transitanschluss (173) der zweiten Festphasenextraktionssäule und verbunden mit oder getrennt von dem Transitanschluss (173) der zweiten Festphasenextraktionssäule; und
einen zweiten Säulenauslassanschluss (176), angeordnet angrenzend an den Verbindungsanschluss der zweiten Probentrennsäule (174) und selektiv verbunden mit dem Verbindungsanschluss der zweiten Probentrennsäule (174) und dem dritten Auslassanschluss (175) ,
wobei der Schritt (B) umfasst:
in einem Zustand, in dem der erste Pumpenverbindungsanschluss (116), der erste Verbindungsanschluss der Probenspeicherschleife (114), der zweite Verbindungsanschluss der Probenspeicherschleife (115) und der erste Auslassanschluss (117) verbunden sind, der erste Einlassanschluss (131) und der erste Säulenventilverbindungsanschluss (132) verbunden sind, der Einlassanschluss der ersten Festphasenextraktionssäule (151) und der Verbindungsanschluss der ersten Festphasenextraktionssäule (152) verbunden sind und der Transitanschluss der ersten Festphasenextraktionssäule (153) und der zweite Auslassanschluss (155) verbunden sind, Injizieren der in der Probenspeicherschleife (111) gespeicherten Probe zusammen mit dem von der ersten Pumpe (P1) zugeführten ersten Lösungsmittel auf die erste Festphasenextraktionssäule (SPE1) und Entsalzen und Konzentrieren der Probe, und
in einem Zustand, in dem der zweite Pumpenverbindungsanschluss (134) und der zweite Säulenventilverbindungsanschluss (133) verbunden sind, der Einlassanschluss der zweiten Festphasenextraktionssäule (171) und der Transitanschluss der zweiten Festphasenextraktionssäule (173) verbunden sind, und der Verbindungsanschluss der zweiten Festphasenextraktionssäule (172) und der Verbindungsanschluss der zweiten Probentrennsäule (174) verbunden sind, Injizieren des ersten Lösungsmittels durch die zweite Pumpe (P2) auf die zweite Festphasenextraktionssäule (SPE2) und die zweite Probentrennsäule (COL2), um die zweite Festphasenextraktionssäule (SPE2) und die zweite Probentrennsäule (COL2) kontinuierlich zu equilibrieren.

4. Verfahren zum Steuern einer voneinander unabhängigen dualen Online-Flüssigkeitschromatographievorrichtung nach Anspruch 3, wobei der Schritt (C) umfasst,
in einem Zustand, in dem der erste Pumpenverbindungsanschluss (116) und der erste Auslassanschluss (117) verbunden sind, der erste Einlassanschluss (131) und der erste Säulenventilverbindungsanschluss (132) verbunden sind, der Einlassanschluss der ersten Festphasenextraktionssäule (151) und der Transitanschluss der ersten Festphasenextraktionssäule (153) verbunden sind und der Verbindungsanschluss der ersten Festphasenextraktionssäule (152) und der Verbindungsanschluss der ersten Probentrennsäule (154) verbunden sind, Injizieren der gemischten Lösung, die das erste Lösungsmittel und das zweite Lösungsmittel enthält, durch die erste Pumpe (P1) auf die erste Festphasenextraktionssäule (SPE1) und die erste Probentrennsäule (COL1), um die isokratische Elution durchzuführen, und
in einem Zustand, in dem der zweite Pumpenverbindungsanschluss (134) und der zweite Säulenventilverbindungsanschluss (133) verbunden sind, der Einlassanschluss der zweiten Festphasenextraktionssäule (171) und der Transitanschluss der zweiten Festphasenextraktionssäule (173) verbunden sind, und der Verbindungsanschluss der zweiten Festphasenextraktionssäule (172) und der Verbindungsanschluss der zweiten Probentrennsäule (174) verbunden sind, Injizieren des ersten Lösungsmittels durch die zweite Pumpe (P2) auf die zweite Festphasenextraktionssäule (SPE2) und die zweite Probentrennsäule (COL2), um die zweite Festphasenextraktionssäule (SPE2) und die zweite Probentrennsäule (COL2) kontinuierlich zu equilibrieren.

5. Verfahren zum Steuern einer voneinander unabhängigen dualen Online-Flüssigkeitschromatographievorrichtung nach Anspruch 4, wobei der Schritt (D) umfasst,
in einem Zustand, in dem der zweite Pumpenverbindungsanschluss (134) und der erste Säulenventilverbindungsanschluss (132) verbunden sind, der Einlassanschluss der ersten Festphasenextraktionssäule (151) und der Transitanschluss der ersten Festphasenextraktionssäule (153) verbunden sind, und der Verbindungsanschluss der ersten Festphasenextraktionssäule (152) und der Verbindungsanschluss der ersten Probentrennsäule (154) verbunden sind, Injizieren der gemischten Lösung, die das mit der Zeit zunehmende zweite Lösungsmittel enthält, durch die zweite Pumpe (P2) auf die erste Festphasenextraktionssäule (SPE1) und die erste Probentrennsäule (COL1), um die Gradientenelution zur Trennung und Analyse der Probe durchzuführen, und
gleichzeitig in einem Zustand, in dem der erste Pumpenverbindungsanschluss (116) und der erste Auslassanschluss (117) verbunden sind, der erste Verbindungsanschluss (131) und der zweite Säulenventilverbindungsanschluss (133) verbunden sind, der Einlassanschluss der zweiten Festphasenextraktionssäule (171) und der Transitanschluss der zweiten Festphasenextraktionssäule (173) verbunden sind, und der Verbindungsanschluss der zweiten Festphasenextraktionssäule (172) und der Verbindungsanschluss der zweiten Probentrennsäule (174) verbunden sind, Injizieren des ersten Lösungsmittels durch die erste Pumpe (P1) auf die zweite Festphasenextraktionssäule (SPE2) und die zweite Probentrennsäule (COL2), um die zweite Festphasenextraktionssäule (SPE2) und die zweite Probentrennsäule (COL2) kontinuierlich zu equilibrieren,
wobei der Schritt (E) umfasst,
in einem Zustand, in dem der zweite Pumpenverbindungsanschluss (134) und der erste Säulenventilverbindungsanschluss (132) verbunden sind, der Einlassanschluss der ersten Festphasenextraktionssäule (151) und der Transitanschluss der ersten Festphasenextraktionssäule (153) verbunden sind, und der Verbindungsanschluss der ersten Festphasenextraktionssäule (152) und der Verbindungsanschluss der ersten Probentrennsäule (154) verbunden sind, Injizieren der gemischten Lösung, die das mit der Zeit zunehmende zweite Lösungsmittel enthält, durch die zweite Pumpe (P2) auf die erste Festphasenextraktionssäule (SPE1) und die erste Probentrennsäule (COL1), um eine Gradientenelution zur Trennung und Analyse der Probe durchzuführen, gleichzeitig, in einem Zustand, in dem der erste Verbindungsanschluss der Probenspeicherschleife (114) und der zweite Verbindungsanschluss der Probenspeicherschleife (115) mit dem Einlassanschluss der Probe (112) bzw. dem Auslassanschluss der Probe (113) verbunden sind, Empfangen der Probe vom Probeninjektor (S) und Injizieren und Speichern der Probe in der Probenspeicherschleife (111), und
gleichzeitig in einem Zustand, in dem der erste Pumpenverbindungsanschluss (116) und der erste Auslassanschluss (117) verbunden sind, der erste Einlassanschluss (131) und der zweite Säulenventilverbindungsanschluss (133) verbunden sind, der Einlassanschluss der zweiten Festphasenextraktionssäule (171) und der Transitanschluss der zweiten Festphasenextraktionssäule (173) verbunden sind, und der Verbindungsanschluss der zweiten Festphasenextraktionssäule (172) und der Verbindungsanschluss der zweiten Probentrennsäule (174) verbunden sind, Injizieren des ersten Lösungsmittels durch die erste Pumpe (P1) auf die zweite Festphasenextraktionssäule (SPE2) und die zweite Probentrennsäule (COL2), um die zweite Festphasenextraktionssäule (SPE2) und die zweite Probentrennsäule (COL2) kontinuierlich zu equilibrieren,
wobei der Schritt (F) umfasst,
in einem Zustand, in dem der zweite Pumpenverbindungsanschluss (134) und der erste Säulenventilverbindungsanschluss (132) verbunden sind, der Einlassanschluss der ersten Festphasenextraktionssäule (151) und der Transitanschluss der ersten Festphasenextraktionssäule (153) verbunden sind, und der Verbindungsanschluss der ersten Festphasenextraktionssäule (152) und der Verbindungsanschluss der ersten Probentrennsäule (154) verbunden sind, Zuführen der gemischten Lösung, die das mit der Zeit zunehmende zweite Lösungsmittel enthält, durch die zweite Pumpe (P2) auf die erste Festphasenextraktionssäule (SPE1) und die erste Probentrennsäule (COL1), um eine Gradientenelution zur Trennung und Analyse der Probe durchzuführen, und gleichzeitig, in einem Zustand, in dem der erste Pumpenverbindungsanschluss (116), der erste Verbindungsanschluss der Probenspeicherschleife (114), der zweite Verbindungsanschluss der Probenspeicherschleife (115) und der erste Auslassanschluss (117) verbunden sind, der erste Einlassanschluss (131) und der zweite Säulenventilverbindungsanschluss (133) verbunden sind, der Einlassanschluss der zweiten Festphasenextraktionssäule (171) und der Verbindungsanschluss der zweiten Festphasenextraktionssäule (172) verbunden sind und der Transitanschluss der zweiten Festphasenextraktionssäule (173) und der dritte Auslassanschluss (175) verbunden sind, Injizieren der in der Probenspeicherschleife (111) gespeicherten Probe zusammen mit dem von der ersten Pumpe (P1) zugeführten ersten Lösungsmittel auf die zweite Festphasenextraktionssäule (SPE2) und Entsalzten und Konzentrieren der Probe, und
wobei der Schritt (G) umfasst
in einem Zustand, in dem der zweite Pumpenverbindungsanschluss (134) und der erste Säulenventilverbindungsanschluss (132) verbunden sind, der Einlassanschluss der ersten Festphasenextraktionssäule (151) und der Transitanschluss der ersten Festphasenextraktionssäule (153) verbunden sind, und der Verbindungsanschluss der ersten Festphasenextraktionssäule (152) und der Verbindungsanschluss der ersten Probentrennsäule (154) verbunden sind, Injizieren der gemischten Lösung, die das mit der Zeit zunehmende zweite Lösungsmittel enthält, durch die zweite Pumpe (P2) auf die erste Festphasenextraktionssäule (SPE1) und die erste Probentrennsäule (COL1), um die Gradientenelution zur Trennung und Analyse der Probe durchzuführen, und gleichzeitig, in einem Zustand, in dem der erste Pumpenverbindungsanschluss (116) und der erste Auslassanschluss (117) verbunden sind, der erste Einlassanschluss (131) und der zweite Säulenventilverbindungsanschluss (133) verbunden sind, der Einlassanschluss der zweiten Festphasenextraktionssäule (171) und der Transitanschluss der zweiten Festphasenextraktionssäule (173) verbunden sind, und der Verbindungsanschluss der zweiten Festphasenextraktionssäule (172) und der Verbindungsanschluss der zweiten Probentrennsäule (174) verbunden sind, Injizieren der gemischten Lösung, die das erste Lösungsmittel und das zweite Lösungsmittel enthält, durch die erste Pumpe (P1) auf die zweite Festphasenextraktionssäule (SPE2) und die zweite Probentrennsäule (COL2), um die isokratische Elution durchzuführen.

6. Verfahren zum Steuern einer voneinander unabhängigen dualen Online-Flüssigkeitschromatographievorrichtung nach Anspruch 5, wobei wenn Schritt (I) umfasst,
in einem Zustand, in dem der zweite Pumpenverbindungsanschluss (134) und der zweite Säulenventilverbindungsanschluss (133) verbunden sind, der Einlassanschluss der zweiten Festphasenextraktionssäule (171) und der Transitanschluss der zweiten Festphasenextraktionssäule (173) verbunden sind, und der Verbindungsanschluss der zweiten Festphasenextraktionssäule (172) und der Verbindungsanschluss der zweiten Probentrennsäule (174) verbunden sind, Zuführen der gemischten Lösung, die das mit der Zeit zunehmende zweite Lösungsmittel enthält, durch die zweite Pumpe (P2) auf die zweite Festphasenextraktionssäule (SPE2) und die zweite Probentrennsäule (COL2), um die Gradientenelution zur Trennung und Analyse der Probe durchzuführen, und
gleichzeitig in einem Zustand, in dem der erste Pumpenverbindungsanschluss (116) und der erste Auslassanschluss (117) verbunden sind, der erste Einlassanschluss (131) und der erste Säulenventilverbindungsanschluss (132) verbunden sind, der Einlassanschluss der ersten Festphasenextraktionssäule (151) und der Transitanschluss der ersten Festphasenextraktionssäule (153) verbunden sind, und der Verbindungsanschluss der ersten Festphasenextraktionssäule (152) und der Verbindungsanschluss der ersten Probentrennsäule (154) verbunden sind, Injizieren der gemischten Lösung, die das erste Lösungsmittel und das zweite Lösungsmittel enthält, durch die erste Pumpe (P1) auf die erste Festphasenextraktionssäule (SPE1) und die erste Probentrennsäule (COL1) injiziert wird, um die erste Festphasenextraktionssäule (SPE1) und die erste Probentrennsäule (COL1) zu waschen,
wobei der Schritt (J) umfasst,
in einem Zustand, in dem der zweite Pumpenverbindungsanschluss (134) und der zweite Säulenventilverbindungsanschluss (133) verbunden sind, der Einlassanschluss der zweiten Festphasenextraktionssäule (171) und der Transitanschluss der zweiten Festphasenextraktionssäule (173) verbunden sind, und der Verbindungsanschluss der zweiten Festphasenextraktionssäule (172) und der Verbindungsanschluss der zweiten Probentrennsäule (174) verbunden sind, Zuführen der gemischten Lösung, die das mit der Zeit zunehmende zweite Lösungsmittel enthält, durch die zweite Pumpe (P2) in die zweite Festphasenextraktionssäule (SPE2) und die zweite Probentrennsäule (COL2), um die Gradientenelution zur Trennung und Analyse der Probe durchzuführen, und
gleichzeitig, in einem Zustand, in dem der erste Pumpenverbindungsanschluss (116) und der erste Auslassanschluss (117) verbunden sind, der erste Einlassanschluss (131) und der erste Säulenventilverbindungsanschluss (132) verbunden sind, der Einlassanschluss der ersten Festphasenextraktionssäule (151) und der Transitanschluss der ersten Festphasenextraktionssäule (153) verbunden sind, und der Verbindungsanschluss der ersten Festphasenextraktionssäule (152) und der Verbindungsanschluss der ersten Probentrennsäule (154) verbunden sind, Injizieren des ersten Lösungsmittels durch die erste Pumpe (P1), auf die erste Festphasenextraktionssäule (SPE1) und die erste Probentrennsäule (COL1), um die erste Festphasenextraktionssäule (SPE1) und die erste Probentrennsäule (COL1) zu equilibrieren,
wobei der Schritt (K) umfasst,
in einem Zustand, in dem der zweite Pumpenverbindungsanschluss (134) und der zweite Säulenventilverbindungsanschluss (133) verbunden sind, der Einlassanschluss der zweiten Festphasenextraktionssäule (171) und der Transitanschluss der zweiten Festphasenextraktionssäule (173) verbunden sind, und der Verbindungsanschluss der zweiten Festphasenextraktionssäule (172) und der Verbindungsanschluss der zweiten Probentrennsäule (174) verbunden sind, Injizieren der gemischten Lösung, die das mit der Zeit zunehmende zweite Lösungsmittel enthält, durch die zweite Pumpe (P2) auf die zweite Festphasenextraktionssäule (SPE2) und die zweite Probentrennsäule (COL2), um die Gradientenelution zur Trennung und Analyse der Probe durchzuführen,
gleichzeitig, in einem Zustand, in dem der erste Verbindungsanschluss der Probenspeicherschleife (114) und der zweite Verbindungsanschluss der Probenspeicherschleife (115) mit dem Probeneinlassanschluss (112) bzw. dem Probenauslassanschluss (113) verbunden sind, Empfangen der Probe vom Probeninjektor (S) und Injizieren und Speichern der Probe in der Probenspeicherschleife (111), und
gleichzeitig, in einem Zustand, in dem der erste Pumpenverbindungsanschluss (116) und der erste Auslassanschluss (117) verbunden sind, der erste Einlassanschluss (131) und der erste Säulenventilanschluss (132) verbunden sind, der Einlassanschluss der ersten Festphasenextraktionssäule (151) und der Transitanschluss der ersten Festphasenextraktionssäule (153) verbunden sind, und der Verbindungsanschluss der ersten Festphasenextraktionssäule (152) und der Verbindungsanschluss der ersten Probentrennsäule (154) verbunden sind, Injizieren des ersten Lösungsmittels durch die erste Pumpe (P1) auf die erste Festphasenextraktionssäule (SPE1) und die erste Probentrennsäule (COL1), um die erste Festphasenextraktionssäule (SPE1) und die erste Probentrennsäule (COL1) kontinuierlich zu equilibrieren,
wobei der Schritt (L) umfasst,
in einem Zustand, in dem der zweite Pumpenverbindungsanschluss (134) und der zweite Säulenventilverbindungsanschluss (133) verbunden sind, der Einlassanschluss der zweiten Festphasenextraktionssäule (171) und der Transitanschluss der zweiten Festphasenextraktionssäule (173) verbunden sind, und der Verbindungsanschluss der zweiten Festphasenextraktionssäule (172) und der Verbindungsanschluss der zweiten Probentrennsäule (174) verbunden werden, Injizieren der gemischten Lösung, die das mit der Zeit zunehmende zweite Lösungsmittel enthält, durch die zweite Pumpe (P2) auf die zweite Festphasenextraktionssäule (SPE2) und die zweite Probentrennsäule (COL2), um die Gradientenelution zur Trennung und Analyse der Probe durchzuführen, und
gleichzeitig, in einem Zustand, in dem der erste Pumpenverbindungsanschluss (116), der erste Verbindungsanschluss der Probenspeicherschleife (114), der zweite Verbindungsanschluss der Probenspeicherschleife (115) und der erste Auslassanschluss (117) verbunden sind, der erste Einlassanschluss (131) und der erste Säulenventilverbindungsanschluss (132) verbunden sind, der Einlassanschluss der ersten Festphasenextraktionssäule (151) und der Verbindungsanschluss der ersten Festphasenextraktionssäule (152) verbunden sind, und der Transitanschluss der ersten Festphasenextraktionssäule (153) und der zweite Auslassanschluss (155) verbunden sind, Injizieren der in der Probenspeicherschleife aufbewahrten Probe zusammen mit dem von der ersten Pumpe zugeführten ersten Lösungsmittel auf die erste Festphasenextraktionssäule und Entsalzen und Konzentrieren der Probe, und
wobei der Schritt (M) umfasst,
in einem Zustand, in dem der zweite Pumpenverbindungsanschluss (134) und der zweite Säulenventilverbindungsanschluss (133) verbunden sind, der Einlassanschluss der zweiten Festphasenextraktionssäule (171) und der Transitanschluss der zweiten Festphasenextraktionssäule (173) verbunden sind, und der Verbindungsanschluss der zweiten Festphasenextraktionssäule (172) und der Verbindungsanschluss der zweiten Probentrennsäule (174) verbunden sind, Injizieren der gemischten Lösung, die das mit der Zeit zunehmende zweite Lösungsmittel enthält, durch die zweite Pumpe (P2) auf die zweite Festphasenextraktionssäule (SPE2) und die zweite Probentrennsäule (COL2), um die Gradientenelution zur Trennung und Analyse der Probe durchzuführen, und
gleichzeitig, in einem Zustand, in dem der erste Pumpenverbindungsanschluss (116) und der erste Auslassanschluss (117) verbunden sind, der erste Einlassanschluss (131) und der erste Säulenventilverbindungsanschluss (132) verbunden sind, der Einlassanschluss der ersten Festphasenextraktionssäule (151) und der Transitanschluss der ersten Festphasenextraktionssäule (153) verbunden sind, und der Verbindungsanschluss der ersten Festphasenextraktionssäule (152) und der Verbindungsanschluss der ersten Probentrennsäule (154) verbunden sind, Zuführen der gemischten Lösung, die das erste Lösungsmittel und das zweite Lösungsmittel enthält, durch die erste Pumpe (P1) auf die erste Festphasenextraktionssäule (SPE1) und die erste Probentrennsäule (COL1), um die isokratische Elution durchzuführen.

7. Verfahren zum Steuern einer voneinander unabhängigen dualen Online-Flüssigchromatographievorrichtung nach Anspruch 5, wobei wenn der Schritt (O) wiederholte Durchführung der Schritte (D) bis (N) in einer sequentiellen Reihenfolge umfasst, der Schritt (D) vor dem Equilibrieren der zweiten Festphasenextraktionssäule (SPE2) und der zweiten Probentrennsäule (COL2), in einem Zustand, in dem der erste Pumpenverbindungsanschluss (116) und der erste Auslassanschluss (117) verbunden sind, und der erste Einlassanschluss (131) und der zweite Säulenventilverbindungsanschluss (133) verbunden sind, Injizieren der gemischten Lösung, die das erste Lösungsmittel und das zweite Lösungsmittel enthält, umfasst, durch die erste Pumpe (P1) auf die zweite Festphasenextraktionssäule (SPE2) und die zweite Probentrennsäule (COL2), um die zweite Festphasenextraktionssäule (SPE2) und die zweite Probentrennsäule (COL2) zu waschen.

8. Verfahren zur Steuerung einer voneinander unabhängigen Dual-Online-Flüssigchromatographievorrichtung nach Anspruch 1, wobei der Schritt (H) Anpassen einer Endzeit der Trennung und Analyse der Probe in der ersten Probentrennsäule (COL1) an eine Zeit unmittelbar vor der Elution eines ersten Peptids aus der zweiten Probentrennsäule (COL2) umfasst.

9. Verfahren zur Steuerung einer voneinander unabhängigen Dual-Online-Flüssigchromatographievorrichtung nach Anspruch 1, wobei der Schritt (N) Anpassen einer Endzeit der Trennung und Analyse der Probe in der zweiten Probentrennsäule (COL2) an eine Zeit unmittelbar vor der Elution eines ersten Peptids aus der ersten Probentrennsäule (COL1) umfasst.

## Revendications

1. Méthode de commande d'un dispositif de chromatographie en phase liquide en ligne double indépendant l'un de l'autre, comprenant :
(A) l'injection, par un injecteur d'échantillon (S) raccordé à une vanne d'entrée d'échantillon (110), d'un échantillon sur une boucle de stockage d'échantillon (111) de la vanne d'entrée d'échantillon (110) pour stocker l'échantillon, et en même temps, l'injection, par une première pompe (P1) raccordée à la vanne d'entrée d'échantillon (110), d'un premier solvant sur une première colonne d'extraction en phase solide (SPE1) et une première colonne de séparation d'échantillon (COL1) d'une première vanne de colonne (150) par l'intermédiaire de la vanne d'entrée d'échantillon (110) et une vanne de sélection de colonne (130) pour équilibrer la première colonne d'extraction en phase solide (SPE1) et la première colonne de séparation d'échantillon (COL1), et l'injection, par une seconde pompe (P2) raccordée à la vanne de sélection de colonne (130), du premier solvant sur une seconde colonne d'extraction en phase solide (SPE2) et une seconde colonne de séparation d'échantillon (COL2) d'une seconde vanne de colonne (170) par l'intermédiaire de la vanne de sélection de colonne (130) pour équilibrer la seconde colonne d'extraction en phase solide (SPE2) et la seconde colonne de séparation d'échantillon (COL2) ;
(B) la réalisation d'une commutation de vanne sur la vanne d'entrée d'échantillon (110) et la première vanne de colonne (150) pour injecter l'échantillon stocké dans la boucle de stockage d'échantillon (111) sur la première colonne d'extraction en phase solide (SPE1) par l'intermédiaire de la vanne de sélection de colonne (130) conjointement avec le premier solvant introduit par la première pompe (P1), et le dessalage et la concentration de l'échantillon, et en même temps, l'injection, par la seconde pompe (P2), du premier solvant sur la seconde colonne d'extraction en phase solide (SPE2) et la seconde colonne de séparation d'échantillon (COL2) par l'intermédiaire de la vanne de sélection de colonne (130) pour équilibrer en continu la seconde colonne d'extraction en phase solide (SPE2) et la seconde colonne de séparation d'échantillon (COL2) ;
(C) la réalisation, de nouveau, d'une commutation de vanne sur la vanne d'entrée d'échantillon (110) et la première vanne de colonne (150), l'injection, par la première pompe (P1), d'une solution mélangée du premier solvant et d'un second solvant sur la première colonne d'extraction en phase solide (SPE1) et la première colonne de séparation d'échantillon (COL1) par l'intermédiaire de la vanne d'entrée d'échantillon (110) et la vanne de sélection de colonne (130) pour effectuer une élution isocratique[fig.5][70], et en même temps, l'injection, par la seconde pompe (P2), du premier solvant sur la seconde colonne d'extraction en phase solide (SPE2) et la seconde colonne de séparation d'échantillon (COL2) par l'intermédiaire de la vanne de sélection de colonne (130) pour équilibrer en continu la seconde colonne d'extraction en phase solide (SPE2) et la seconde colonne de séparation d'échantillon (COL2) ;
(D) réalisation d'une commutation de vanne de la vanne de sélection de colonne (130) pour injecter progressivement, par la seconde pompe (P2), la solution mélangée contenant le second solvant en quantité croissante sur la première colonne d'extraction en phase solide (SPE1) et la première colonne de séparation d'échantillon (COL1) par l'intermédiaire de la vanne de sélection de colonne (130) pour effectuer une élution par gradient en vue de la séparation et de l'analyse de l'échantillon, et en même temps, l'injection, par la première pompe (P1), du premier solvant sur la seconde colonne d'extraction en phase solide (SPE2) et la seconde colonne de séparation d'échantillon (COL2) par l'intermédiaire de la vanne d'entrée d'échantillon (110) et la vanne de sélection de colonne (130) pour équilibrer en continu la seconde colonne d'extraction en phase solide (SPE2) et la seconde colonne de séparation d'échantillon (COL2) ;
(E) pendant la séparation et l'analyse de l'échantillon par l'élution par gradient dans la première colonne de séparation d'échantillon (COL1), l'injection, par l'injecteur d'échantillon (S), de l'échantillon sur la boucle de stockage d'échantillon (111) pour stocker l'échantillon, et en même temps, l'injection, par la première pompe (P1) raccordée à la vanne d'entrée d'échantillon (110), du premier solvant sur la seconde colonne d'extraction en phase solide (SPE2) et la seconde colonne de séparation d'échantillon (COL2) de la seconde vanne de colonne (170) par l'intermédiaire de la vanne d'entrée d'échantillon (110) et la vanne de sélection de colonne (130) pour équilibrer en continu la seconde colonne d'extraction en phase solide (SPE2) et la seconde colonne de séparation d'échantillon (COL2) ;
(F) pendant la séparation et l'analyse de l'échantillon par l'élution par gradient dans la première colonne de séparation d'échantillon (COL1), la réalisation d'une commutation de vanne sur la vanne d'entrée d'échantillon (110) et la seconde vanne de colonne (170), l'injection de l'échantillon stocké dans la boucle de stockage d'échantillon (111) sur la seconde colonne d'extraction en phase solide (SPE2) par l'intermédiaire de la vanne d'entrée d'échantillon (110) et la vanne de sélection de colonne (130) conjointement avec le premier solvant introduit par la première pompe (P1), et le dessalage et la concentration de l'échantillon ;
(G) pendant la séparation et l'analyse de l'échantillon par l'élution par gradient dans la première colonne de séparation d'échantillon (COL1), la réalisation d'une commutation de vanne sur la vanne d'entrée d'échantillon (110) et la seconde vanne de colonne (170) et l'injection, par la première pompe (P1), de la solution mélangée du premier solvant et du second solvant sur la seconde colonne d'extraction en phase solide (SPE2) et la seconde colonne de séparation d'échantillon (COL2) par l'intermédiaire de la vanne d'entrée d'échantillon (110) et la vanne de sélection de colonne (130) pour effectuer une élution isocratique ;
(H) l'achèvement simultané de la séparation et de l'analyse de l'échantillon dans la première colonne de séparation d'échantillon (COL1) et de l'élution isocratique à partir de la seconde colonne d'extraction en phase solide (SPE2) et de la seconde colonne de séparation d'échantillon (COL2) ;
(I) la réalisation d'une commutation de vanne de la vanne de sélection de colonne (130) pour injecter progressivement, par la seconde pompe (P2), la solution mélangée contenant le second solvant en quantité croissante sur la seconde colonne d'extraction en phase solide (SPE2) et la seconde colonne de séparation d'échantillon (COL2) par l'intermédiaire de la vanne de sélection de colonne (130) pour effectuer une élution par gradient en vue de la séparation et de l'analyse de l'échantillon, et en même temps, l'injection, par la première pompe (P1), de la solution mélangée contenant le premier solvant et le second solvant sur la première colonne d'extraction en phase solide (SPE1) et la première colonne de séparation d'échantillon (COL1) par l'intermédiaire de la vanne d'entrée d'échantillon (110) et la vanne de sélection de colonne (130) pour laver la première colonne d'extraction en phase solide (SPE1) et la première colonne de séparation d'échantillon (COL1) ;
(J) pendant la séparation et l'analyse de l'échantillon par l'élution par gradient dans la seconde colonne de séparation d'échantillon (COL2), l'injection, par la première pompe (P1), du premier solvant sur la première colonne d'extraction en phase solide (SPE1) et la première colonne de séparation d'échantillon (COL1) par l'intermédiaire de la vanne d'entrée d'échantillon (110) et la vanne de sélection de colonne (130) pour équilibrer la première colonne d'extraction en phase solide (SPE1) et la première colonne de séparation d'échantillon (COL1) ;
(K) pendant la séparation et l'analyse de l'échantillon par l'élution par gradient dans la seconde colonne de séparation d'échantillon (COL2), l'injection, par l'injecteur d'échantillon (S), de l'échantillon sur la boucle de stockage d'échantillon (111) pour stocker l'échantillon, et en même temps, l'injection, par la première pompe (P1) raccordée à la vanne d'entrée d'échantillon (110), du premier solvant sur la première colonne d'extraction en phase solide (SPE1) et la première colonne de séparation d'échantillon (COL1) de la première vanne de colonne (150) par l'intermédiaire de la vanne d'entrée d'échantillon (110) et la vanne de sélection de colonne (130) pour équilibrer en continu la première colonne d'extraction en phase solide (SPE1) et la première colonne de séparation d'échantillon (COL1) ;
(L) pendant la séparation et l'analyse de l'échantillon par l'élution par gradient dans la seconde colonne de séparation d'échantillon (COL2), la réalisation d'une commutation de vanne sur la vanne d'entrée d'échantillon (110) et la première vanne de colonne (150) pour injecter l'échantillon stocké dans la boucle de stockage d'échantillon (111) sur la première colonne d'extraction en phase solide (SPE1) par l'intermédiaire de la vanne d'entrée d'échantillon (110) et la vanne de sélection de colonne (130) conjointement avec le premier solvant introduit par la première pompe (P1), et le dessalage et la concentration de l'échantillon ;
(M) pendant la séparation et l'analyse de l'échantillon par l'élution par gradient dans la seconde colonne de séparation d'échantillon (COL2), la réalisation d'une commutation de vanne sur la vanne d'entrée d'échantillon (110) et la première vanne de colonne (150), et l'injection, par la première pompe (P1), de la solution mélangée du premier solvant et du second solvant sur la première colonne d'extraction en phase solide (SPE1) et la première colonne de séparation d'échantillon (COL1) par l'intermédiaire de la vanne d'entrée d'échantillon (110) et la vanne de sélection de colonne (130) pour effectuer une élution isocratique ;
(N) l'achèvement simultané de la séparation et de l'analyse de l'échantillon dans la seconde colonne de séparation d'échantillon (COL2) et de l'élution isocratique à partir de la première colonne d'extraction en phase solide (SPE1) et de la première colonne de séparation d'échantillon (COL1) ; et
(O) la réalisation répétée des étapes (D) à (N) dans un ordre séquentiel en fonction du nombre de séparations et analyses de l'échantillon ou du nombre d'échantillons.

2. Méthode de commande d'un dispositif de chromatographie en phase liquide en ligne double indépendant l'un de l'autre selon la revendication 1, dans laquelle la vanne d'entrée d'échantillon (110) inclut :
un orifice d'entrée d'échantillon (112) raccordé à l'injecteur d'échantillon (S) ;
un orifice de sortie d'échantillon (113) disposé de manière adjacente à l'orifice d'entrée d'échantillon (112) ;
un orifice de raccordement (116) de première pompe raccordé à la première pompe (P1) ;
un premier orifice de sortie (117) disposé de manière adjacente à l'orifice de raccordement (116) de première pompe et raccordé à la vanne de sélection de colonne (130) ; et
un premier orifice de raccordement (114) de boucle de stockage d'échantillon et un second orifice de raccordement (115) de boucle de stockage d'échantillon auxquels deux extrémités de la boucle de stockage d'échantillon (111) sont respectivement raccordées,
dans laquelle la vanne de sélection de colonne (130) inclut :
un premier orifice d'entrée (131) raccordé au premier orifice de sortie (117) ;
un orifice de raccordement (132) de première vanne de colonne raccordé à la première vanne de colonne (150) ;
un orifice de raccordement (133) de seconde vanne de colonne raccordé à la seconde vanne de colonne (170) ; et
un orifice de raccordement (134) de seconde pompe raccordé à la seconde pompe (P2), et
dans laquelle l'étape (A) comprend,
dans un état où le premier orifice de raccordement (114) de boucle de stockage d'échantillon et le second orifice de raccordement (115) de boucle de stockage d'échantillon sont respectivement raccordés à l'orifice d'entrée d'échantillon (112) et à l'orifice de sortie d'échantillon (113), la réception de l'échantillon en provenance de l'injecteur d'échantillon (S), et l'injection et le stockage de l'échantillon dans la boucle de stockage d'échantillon (111),
dans un état où l'orifice de raccordement (116) de première pompe, le premier orifice de sortie (117), le premier orifice d'entrée (131) et l'orifice de raccordement (132) de première vanne de colonne sont raccordés, l'introduction, par la première pompe (P1), du premier solvant dans la vanne de sélection de colonne (130), l'introduction du premier solvant introduit dans la vanne de sélection de colonne (130) dans la première vanne de colonne (150) et l'injection sur la première colonne d'extraction en phase solide (SPE1) et la première colonne de séparation d'échantillon (COL1) pour équilibrer la première colonne d'extraction en phase solide (SPE1) et la première colonne de séparation d'échantillon (COL1), et
dans un état où l'orifice de raccordement (134) de seconde pompe et l'orifice de raccordement (133) de seconde vanne de colonne sont raccordés, l'introduction, par la seconde pompe (P2), du premier solvant dans la vanne de sélection de colonne (130), l'introduction du premier solvant introduit dans la vanne de sélection de colonne (130) dans la seconde vanne de colonne (170) et l'injection sur la seconde colonne d'extraction en phase solide (SPE2) et la seconde colonne de séparation d'échantillon (COL2) pour équilibrer la seconde colonne d'extraction en phase solide (SPE2) et la seconde colonne de séparation des échantillons (COL2).

3. Méthode de commande d'un dispositif de chromatographie en phase liquide en ligne double indépendant l'un de l'autre selon la revendication 2, dans laquelle la première vanne de colonne (150) inclut :
un orifice de raccordement (152) de première colonne d'extraction en phase solide et un orifice de transfert (153) de première colonne d'extraction en phase solide raccordés à deux extrémités de la première colonne d'extraction en phase solide (SPE1), respectivement ;
un orifice d'entrée (151) de première colonne d'extraction en phase solide raccordé à l'orifice de raccordement (132) de première vanne de colonne, et raccordé sélectivement à l'orifice de raccordement (152) de première colonne d'extraction en phase solide et à l'orifice de transfert (153) de première colonne d'extraction en phase solide ;
un orifice de raccordement (154) de première colonne de séparation d'échantillon raccordé à la première colonne de séparation d'échantillon (COL1), et raccordé à ou séparé de l'orifice de raccordement (152) de première colonne d'extraction en phase solide ;
un deuxième orifice de sortie (155) disposé de manière adjacente à l'orifice de transfert (153) de première colonne d'extraction en phase solide et raccordé à ou séparé de l'orifice de transfert (153) de première colonne d'extraction en phase solide ; et
un premier orifice de sortie de colonne (156) disposé de manière adjacente à l'orifice de raccordement (154) de première colonne de séparation d'échantillon et raccordé sélectivement à l'orifice de raccordement (154) de première colonne de séparation d'échantillon et au deuxième orifice de sortie (155),
dans laquelle la seconde vanne de colonne (170) inclut :
un orifice de raccordement (172) de seconde colonne d'extraction en phase solide et un orifice de transfert (173) de seconde colonne d'extraction en phase solide raccordés à deux extrémités de la seconde colonne d'extraction en phase solide (SPE2), respectivement ;
un orifice d'entrée (171) de seconde colonne d'extraction en phase solide raccordé à l'orifice de raccordement (133) de seconde vanne de colonne et raccordé sélectivement à l'orifice de raccordement (172) de seconde colonne d'extraction en phase solide et à l'orifice de transfert (173) de seconde colonne d'extraction en phase solide ;
un orifice de raccordement (174) de seconde colonne de séparation d'échantillon raccordé à la seconde colonne de séparation d'échantillon (COL2), et raccordé à ou séparé de l'orifice de raccordement (172) de seconde colonne d'extraction en phase solide ;
un troisième orifice de sortie (175) disposé de manière adjacente à l'orifice de transfert (173) de seconde colonne d'extraction en phase solide et raccordé à ou séparé de l'orifice de transfert (173) de seconde colonne d'extraction en phase solide ; et
un deuxième orifice de sortie de colonne (176) disposé de manière adjacente à l'orifice de raccordement (174) de seconde colonne de séparation d'échantillon et raccordé sélectivement à l'orifice de raccordement (174) de seconde colonne de séparation d'échantillon et au troisième orifice de sortie (175),
dans laquelle l'étape (B) comprend,
dans un état où l'orifice de raccordement (116) de première pompe, le premier orifice de raccordement (114) de boucle de stockage d'échantillon, le second orifice de raccordement (115) de boucle de stockage d'échantillon et le premier orifice de sortie (117) sont raccordés, le premier orifice d'entrée (131) et l'orifice de raccordement (132) de première vanne de colonne sont raccordés, l'orifice d'entrée (151) de première colonne d'extraction en phase solide et l'orifice de raccordement (152) de première colonne d'extraction en phase solide sont raccordés et l'orifice de transfert (153) de première colonne d'extraction en phase solide et le deuxième orifice de sortie (155) sont raccordés, l'injection de l'échantillon stocké dans la boucle de stockage d'échantillon (111) sur la première colonne d'extraction en phase solide (SPE1) conjointement avec le premier solvant introduit par la première pompe (P1), et le dessalage et le concentration de l'échantillon, et
dans un état où l'orifice de raccordement (134) de seconde pompe et l'orifice de raccordement (133) de seconde vanne de colonne sont raccordés, l'orifice d'entrée (171) de seconde colonne d'extraction en phase solide et l'orifice de transfert (173) de seconde colonne d'extraction en phase solide sont raccordés et l'orifice de raccordement (172) de seconde colonne d'extraction en phase solide et l'orifice de raccordement (174) de seconde colonne de séparation d'échantillon sont raccordés, l'injection, par la seconde pompe (P2), du premier solvant sur la seconde colonne d'extraction en phase solide (SPE2) et la seconde colonne de séparation d'échantillon (COL2) pour équilibrer en continu la seconde colonne d'extraction en phase solide (SPE2) et la seconde colonne de séparation d'échantillon (COL2).

4. Méthode de commande d'un dispositif de chromatographie en phase liquide en ligne double indépendant l'un de l'autre selon la revendication 3, dans laquelle l'étape (C) comprend,
dans un état où l'orifice de raccordement (116) de première pompe et le premier orifice de sortie (117) sont raccordés, le premier orifice d'entrée (131) et l'orifice de raccordement (132) de première vanne de colonne sont raccordés, l'orifice d'entrée (151) de première colonne d'extraction en phase solide et l'orifice de transfert (153) de première colonne d'extraction en phase solide sont raccordés et l'orifice de raccordement (152) de première colonne d'extraction en phase solide et l'orifice de raccordement (154) de première colonne de séparation d'échantillon sont raccordés, l'injection, par la première pompe (P1), de la solution mélangée contenant le premier solvant et le second solvant sur la première colonne d'extraction en phase solide (SPE1) et la première colonne de séparation d'échantillon (COL1) pour effectuer l'élution isocratique, et
dans un état où l'orifice de raccordement (134) de seconde pompe et l'orifice de raccordement (133) de seconde vanne de colonne sont raccordés, l'orifice d'entrée (171) de seconde colonne d'extraction en phase solide et l'orifice de transfert (173) de seconde colonne d'extraction en phase solide sont raccordés, et l'orifice de raccordement (172) de seconde colonne d'extraction en phase solide et l'orifice de raccordement (174) de seconde colonne de séparation d'échantillon sont raccordés, l'injection, par la seconde pompe (P2), du premier solvant sur la seconde colonne d'extraction en phase solide (SPE2) et la seconde colonne de séparation d'échantillon (COL2) pour équilibrer en continu la seconde colonne d'extraction en phase solide (SPE2) et la seconde colonne de séparation d'échantillon (COL2).

5. Méthode de commande d'un dispositif de chromatographie en phase liquide en ligne double indépendant l'un de l'autre selon la revendication 4, dans laquelle l'étape (D) comprend,
dans un état où l'orifice de raccordement (134) de seconde pompe et l'orifice de raccordement (132) de première vanne de colonne sont raccordés, l'orifice d'entrée (151) de première colonne d'extraction en phase solide et l'orifice de transfert (153) de première colonne d'extraction en phase solide sont raccordés et l'orifice de raccordement (152) de première colonne d'extraction en phase solide et l'orifice de raccordement (154) de première colonne de séparation d'échantillon sont raccordés, l'injection progressive, par la seconde pompe (P2), de la solution mélangée contenant le second solvant en quantité croissante sur la première colonne d'extraction en phase solide (SPE1) et la première colonne de séparation d'échantillon (COL1) pour effectuer l'élution par gradient en vue de la séparation et de l'analyse de l'échantillon, et
en même temps, dans un état où l'orifice de raccordement (116) de première pompe et le premier orifice de sortie (117) sont raccordés, le premier orifice d'entrée (131) et l'orifice de raccordement (133) de seconde vanne de colonne sont raccordés, l'orifice d'entrée (171) de seconde colonne d'extraction en phase solide et l'orifice de transfert (173) de seconde colonne d'extraction en phase solide sont raccordés et l'orifice de raccordement (172) de seconde colonne d'extraction en phase solide et l'orifice de raccordement (174) de seconde colonne de séparation d'échantillon sont raccordés, l'injection, par la première pompe (P1), du premier solvant sur la seconde colonne d'extraction en phase solide (SPE2) et la seconde colonne de séparation d'échantillon (COL2) pour équilibrer en continu la seconde colonne d'extraction en phase solide (SPE2) et la seconde colonne de séparation d'échantillon (COL2),
dans laquelle l'étape (E) comprend,
dans un état où l'orifice de raccordement (134) de seconde pompe et l'orifice de raccordement (132) de première vanne de colonne sont raccordés, l'orifice d'entrée (151) de première colonne d'extraction en phase solide et l'orifice de transfert (153) de première colonne d'extraction en phase solide sont raccordés et l'orifice de raccordement (152) de première colonne d'extraction en phase solide et l'orifice de raccordement (154) de première colonne de séparation d'échantillon sont raccordés, l'injection progressive, par la seconde pompe (P2), de la solution mélangée contenant le second solvant en quantité croissante sur la première colonne d'extraction en phase solide (SPE1) et la première colonne de séparation d'échantillon (COL1) pour effectuer une élution par gradient en vue de la séparation et de l'analyse de l'échantillon,
en même temps, dans un état où le premier orifice de raccordement (114) de boucle de stockage d'échantillon et le second orifice de raccordement (115) de boucle de stockage d'échantillon sont raccordés respectivement à l'orifice d'entrée d'échantillon (112) et à l'orifice de sortie d'échantillon (113), la réception de l'échantillon en provenance de l'injecteur d'échantillon (S), et l'injection et le stockage de l'échantillon dans la boucle de stockage d'échantillon (111), et
en même temps, dans un état où l'orifice de raccordement (116) de première pompe et le premier orifice de sortie (117) sont raccordés, le premier orifice d'entrée (131) et l'orifice de raccordement (133) de seconde vanne de colonne sont raccordés, l'orifice d'entrée (171) de seconde colonne d'extraction en phase solide et l'orifice de transfert (173) de seconde colonne d'extraction en phase solide sont raccordés et l'orifice de raccordement (172) de seconde colonne d'extraction en phase solide et l'orifice de raccordement (174) de seconde colonne de séparation d'échantillon sont raccordés, l'injection, par la première pompe (P1), du premier solvant sur la seconde colonne d'extraction en phase solide (SPE2) et la seconde colonne de séparation d'échantillon (COL2) pour équilibrer en continu la seconde colonne d'extraction en phase solide (SPE2) et la seconde colonne de séparation d'échantillon (COL2),
dans laquelle l'étape (F) comprend,
dans un état où l'orifice de raccordement (134) de seconde pompe et l'orifice de raccordement (132) de première vanne de colonne sont raccordés, l'orifice d'entrée (151) de première colonne d'extraction en phase solide et l'orifice de transfert (153) de première colonne d'extraction en phase solide sont raccordés et l'orifice de raccordement (152) de première colonne d'extraction en phase solide et l'orifice de raccordement (154) de première colonne de séparation d'échantillon sont raccordés, l'introduction progressive, par la seconde pompe (P2), de la solution mélangée contenant le second solvant en quantité croissante sur la première colonne d'extraction en phase solide (SPE1) et la première colonne de séparation d'échantillon (COL1) pour effectuer une élution par gradient en vue de la séparation et de l'analyse de l'échantillon[81][78], et en même temps, dans un état où l'orifice de raccordement (116) de première pompe, le premier orifice de raccordement (114) de boucle de stockage d'échantillon, le second orifice de raccordement (115) de boucle de stockage d'échantillon et le premier orifice de sortie (117) sont raccordés, le premier orifice d'entrée (131) et l'orifice de raccordement (133) de seconde vanne de colonne sont raccordés, l'orifice d'entrée (171) de seconde colonne d'extraction en phase solide et l'orifice de raccordement (172) de seconde colonne d'extraction en phase solide sont raccordés et l'orifice de transfert (173) de seconde colonne d'extraction en phase solide et le troisième orifice de sortie (175) sont raccordés,l'injection de l'échantillon stocké dans la boucle de stockage d'échantillon (111) sur la seconde colonne d'extraction en phase solide (SPE2) conjointement avec le premier solvant introduit par la première pompe (P1) et le dessalage et la concentration de l'échantillon, et
dans laquelle l'étape (G) comprend,
dans un état où l'orifice de raccordement (134) de seconde pompe et l'orifice de raccordement (132) de première vanne de colonne sont raccordés, l'orifice d'entrée (151) de première colonne d'extraction en phase solide et l'orifice de transfert (153) de première colonne d'extraction en phase solide sont raccordés et l'orifice de raccordement (152) de première colonne d'extraction en phase solide et l'orifice de raccordement (154) de première colonne de séparation d'échantillon sont raccordés, l'injection progressive, par la seconde pompe (P2), de la solution mélangée contenant le second solvant en quantité croissante sur la première colonne d'extraction en phase solide (SPE1) et la première colonne de séparation d'échantillon (COL1) pour effectuer l'élution par gradient en vue de la séparation et de l'analyse de l'échantillon[83] [78], et en même temps, dans un état où l'orifice de raccordement (116) de première pompe et le premier orifice de sortie (117) sont raccordés, le premier orifice d'entrée (131) et l'orifice de raccordement (133) de seconde vanne de colonne sont raccordés, l'orifice d'entrée (171) de seconde colonne d'extraction en phase solide et l'orifice de transfert (173) de seconde colonne d'extraction en phase solide sont raccordés et l'orifice de raccordement (172) de seconde colonne d'extraction en phase solide et l'orifice de raccordement (174) de seconde colonne de séparation d'échantillon sont raccordés, l'injection, par la première pompe (P1), de la solution mélangée contenant le premier solvant et le second solvant sur la seconde colonne d'extraction en phase solide (SPE2) et la seconde colonne de séparation d'échantillon (COL2) pour réaliser l'élution isocratique.

6. Méthode de commande d'un dispositif de chromatographie en phase liquide en ligne double indépendant l'un de l'autre selon la revendication 5, dans laquelle lorsque l'étape (I) comprend,
dans un état où l'orifice de raccordement (134) de seconde pompe et l'orifice de raccordement (133) de seconde vanne de colonne sont raccordés, l'orifice d'entrée (171) de seconde colonne d'extraction en phase solide et l'orifice de transfert (173) de seconde colonne d'extraction en phase solide sont raccordés et l'orifice de raccordement (172) de seconde colonne d'extraction en phase solide et l'orifice de raccordement (174) de seconde colonne de séparation d'échantillon sont raccordés, l'introduction progressive, par la seconde pompe (P2), de la solution mélangée contenant le second solvant en quantité croissante dans la seconde colonne d'extraction en phase solide (SPE2) et la seconde colonne de séparation d'échantillon (COL2) pour effectuer l'élution par gradient en vue de la séparation et de l'analyse de l'échantillon, et
en même temps, dans un état où l'orifice de raccordement (116) de première pompe et le premier orifice de sortie (117) sont raccordés, le premier orifice d'entrée (131) et l'orifice de raccordement (132) de première vanne de colonne sont raccordés, l'orifice d'entrée (151) de première colonne d'extraction en phase solide et l'orifice de transfert (153) de première colonne d'extraction en phase solide sont raccordés et l'orifice de raccordement (152) de première colonne d'extraction en phase solide et l'orifice de raccordement (154) de première colonne de séparation d'échantillon sont raccordés, l'injection, par la première pompe (P1), de la solution mélangée contenant le premier solvant et le second solvant sur la première colonne d'extraction en phase solide (SPE1) et la première colonne de séparation d'échantillon (COL1) pour laver la première colonne d'extraction en phase solide (SPE1) et la première colonne de séparation d'échantillon (COL1),
dans laquelle l'étape (J) comprend,
dans un état où l'orifice de raccordement (134) de seconde pompe et l'orifice de raccordement (133) de seconde vanne de colonne sont raccordés, l'orifice d'entrée (171) de seconde colonne d'extraction en phase solide et l'orifice de transfert (173) de seconde colonne d'extraction en phase solide sont raccordés et l'orifice de raccordement (172) de seconde colonne d'extraction en phase solide et l'orifice de raccordement (174) de seconde colonne de séparation d'échantillon sont raccordés, l'introduction progressive, par la seconde pompe (P2), de la solution mélangée contenant le second solvant en quantité croissante dans la seconde colonne d'extraction en phase solide (SPE2) et la seconde colonne de séparation d'échantillon (COL2) pour effectuer l'élution par gradient en vue de la séparation et de l'analyse de l'échantillon, et
en même temps, dans un état où l'orifice de raccordement (116) de première pompe et le premier orifice de sortie (117) sont raccordés, le premier orifice d'entrée (131) et l'orifice de raccordement (132) de première vanne de colonne sont raccordés, l'orifice d'entrée (151) de première colonne d'extraction en phase solide et l'orifice de transfert (153) de première colonne d'extraction en phase solide sont raccordés et l'orifice de raccordement (152) de première colonne d'extraction en phase solide et l'orifice de raccordement (154) de première colonne de séparation d'échantillon sont raccordés, l'injection, par la première pompe (P1), du premier solvant sur la première colonne d'extraction en phase solide (SPE1) et la première colonne de séparation d'échantillon (COL1) pour équilibrer la première colonne d'extraction en phase solide (SPE1) et la première colonne de séparation d'échantillon (COL1),
dans laquelle l'étape (K) comprend,
dans un état où l'orifice de raccordement (134) de seconde pompe et l'orifice de raccordement (133) de seconde vanne de colonne sont raccordés, l'orifice d'entrée (171) de seconde colonne d'extraction en phase solide et l'orifice de transfert (173) de seconde colonne d'extraction en phase solide sont raccordés et l'orifice de raccordement (172) de seconde colonne d'extraction en phase solide et l'orifice de raccordement (174) de seconde colonne de séparation d'échantillon sont raccordés, l'injection progressive, par la seconde pompe (P2), de la solution mélangée contenant le second solvant en quantité croissante sur la seconde colonne d'extraction en phase solide (SPE2) et la seconde colonne de séparation d'échantillon (COL2) pour effectuer l'élution par gradient en vue de la séparation et de l'analyse de l'échantillon,
en même temps, dans un état où le premier orifice de raccordement (114) de boucle de stockage d'échantillon et le second orifice de raccordement (115) de boucle de stockage d'échantillon sont raccordés respectivement à l'orifice d'entrée d'échantillon (112) et à l'orifice de sortie d'échantillon (113), la réception de l'échantillon en provenance de l'injecteur d'échantillon (S), et l'injection et le stockage de l'échantillon dans la boucle de stockage d'échantillon (111), et
en même temps, dans un état où l'orifice de raccordement (116) de première pompe et le premier orifice de sortie (117) sont raccordés, le premier orifice d'entrée (131) et l'orifice de raccordement (132) de première vanne de colonne sont raccordés, l'orifice d'entrée (151) de première colonne d'extraction en phase solide et l'orifice de transfert (153) de première colonne d'extraction en phase solide sont raccordés et l'orifice de raccordement (152) de première colonne d'extraction en phase solide et l'orifice de raccordement (154) de première colonne de séparation d'échantillon sont raccordés, l'injection, par la première pompe (P1), du premier solvant sur la première colonne d'extraction en phase solide (SPE1) et la première colonne de séparation d'échantillon (COL1) pour équilibrer en continu la première colonne d'extraction en phase solide (SPE1) et la première colonne de séparation d'échantillon (COL1),
dans laquelle l'étape (L) comprend,
dans un état où l'orifice de raccordement (134) de seconde pompe et l'orifice de raccordement (133) de seconde vanne de colonne sont raccordés, l'orifice d'entrée (171) de seconde colonne d'extraction en phase solide et l'orifice de transfert (173) de seconde colonne d'extraction en phase solide sont raccordés et l'orifice de raccordement (172) de seconde colonne d'extraction en phase solide et l'orifice de raccordement (174) de seconde colonne de séparation d'échantillon sont raccordés, l'injection progressive, par la seconde pompe (P2), de la solution mélangée contenant le second solvant en quantité croissante sur la seconde colonne d'extraction en phase solide (SPE2) et la seconde colonne de séparation d'échantillon (COL2) pour effectuer l'élution par gradient en vue de la séparation et de l'analyse de l'échantillon, et
en même temps, dans un état où l'orifice de raccordement (116) de première pompe, le premier orifice de raccordement (114) de boucle de stockage d'échantillon, le second orifice de raccordement (115) de boucle de stockage d'échantillon et le premier orifice de sortie (117) sont raccordés, le premier orifice d'entrée (131) et l'orifice de raccordement (132) de première vanne de colonne sont raccordés, l'orifice d'entrée (151) de première colonne d'extraction en phase solide et l'orifice de raccordement (152) de première colonne d'extraction en phase solide sont raccordés et l'orifice de transfert (153) de première colonne d'extraction en phase solide et le deuxième orifice de sortie (155) sont raccordés, l'injection de l'échantillon stocké dans la boucle de stockage d'échantillon sur la première colonne d'extraction en phase solide conjointement avec le premier solvant introduit par la première pompe, et le dessalage et la concentration de l'échantillon, et
dans laquelle l'étape (M) comprend,
dans un état où l'orifice de raccordement (134) de seconde pompe et l'orifice de raccordement (133) de seconde vanne de colonne sont raccordés, l'orifice d'entrée (171) de seconde colonne d'extraction en phase solide et l'orifice de transfert (173) de seconde colonne d'extraction en phase solide sont raccordés et l'orifice de raccordement (172) de seconde colonne d'extraction en phase solide et l'orifice de raccordement (174) de seconde colonne de séparation d'échantillon sont raccordés, l'injection progressive, par la seconde pompe (P2), de la solution mélangée contenant le second solvant en quantité croissante sur la seconde colonne d'extraction en phase solide (SPE2) et la seconde colonne de séparation d'échantillon (COL2) pour effectuer l'élution par gradient en vue de la séparation et de l'analyse de l'échantillon, et
en même temps, dans un état où l'orifice de raccordement (116) de première pompe et le premier orifice de sortie (117) sont raccordés, le premier orifice d'entrée (131) et l'orifice de raccordement (132) de première vanne de colonne sont raccordés, l'orifice d'entrée (151) de première colonne d'extraction en phase solide et l'orifice de transfert (153) de première colonne d'extraction en phase solide sont raccordés et l'orifice de raccordement (152) de première colonne d'extraction en phase solide et l'orifice de raccordement (154) de première colonne de séparation d'échantillon sont raccordés, l'introduction, par la première pompe (P1), de la solution mélangée contenant le premier solvant et le second solvant sur la première colonne d'extraction en phase solide (SPE1) et la première colonne de séparation d'échantillon (COL1) pour effectuer l'élution isocratique.

7. Méthode de commande d'un dispositif de chromatographie en phase liquide en ligne double indépendant l'un de l'autre selon la revendication 5, dans laquelle lorsque l'étape (O) comprend la réalisation répétée des étapes (D) à (N) dans un ordre séquentiel, l'étape (D) comprend, avant d'équilibrer la seconde colonne d'extraction en phase solide (SPE2) et la seconde colonne de séparation d'échantillon (COL2), dans un état où l'orifice de raccordement (116) de première pompe et le premier orifice de sortie (117) sont raccordés et le premier orifice d'entrée (131) et l'orifice de raccordement (133) de seconde vanne de colonne sont raccordés, l'injection, par la première pompe (P1), de la solution mélangée contenant le premier solvant et le second solvant sur la seconde colonne d'extraction en phase solide (SPE2) et la seconde colonne de séparation d'échantillon (COL2) pour laver la seconde colonne d'extraction en phase solide (SPE2) et la seconde colonne de séparation d'échantillon (COL2).

8. Méthode de commande d'un dispositif de chromatographie en phase liquide en ligne double indépendant l'un de l'autre selon la revendication 1, dans laquelle l'étape (H) comprend la mise en correspondance d'un temps de fin de la séparation et de l'analyse de l'échantillon dans la première colonne de séparation d'échantillon (COL1) avec un temps immédiatement avant qu'un premier peptide ne soit élué à partir de la seconde colonne de séparation d'échantillon (COL2).

9. Méthode de commande d'un dispositif de chromatographie en phase liquide en ligne double indépendant l'un de l'autre selon la revendication 1, dans laquelle l'étape (N) comprend la mise en correspondance d'un temps de fin de la séparation et de l'analyse de l'échantillon dans la seconde colonne de séparation d'échantillon (COL2) avec un temps immédiatement avant qu'un premier peptide ne soit élué à partir de la première colonne de séparation d'échantillon (COL1).
